# EUROPEAN PATENT APPLICATION

(11) **EP 3 822 346 A1**
(43) Date of publication of application: **19.05.2021**
(21) Application number: 19209128.8
(22) Date of filing: 14.11.2019
(51) Int. Cl.: C12N 7/02, C12N 15/86

(54) **USE OF ENGINEERED PACKAGING CELL LINES FOR PRODUCING RECOMBINANT VIRUS PARTICLES**

(71) Applicant: Technische Universität Dresden, 01069 Dresden (DE)
(72) Inventor: LINDEMANN, Dirk, 01239 Dresden (DE)
(74) Representative: PATERIS Patentanwälte PartmbB

(57) **Abstract**

Use of engineered packaging cell lines for producing recombinant virus particles

The invention discloses the use of an engineered mammalian packaging cell line for producing recombinant virus particles, wherein the cell line is engineered to lack cell surface expression of heparan sulfate. Further disclosed are a method for producing recombinant virus particles and a recombinant virus particle obtainable by the method. Further disclosed is a mammalian packaging cell line deposited under number DSM ACC3355 or DSM ACC3356.

## Description

### Field of the invention

The present invention relates to the use of an engineered mammalian packaging cell line for producing recombinant virus particles, wherein the cell line is engineered to lack cell surface expression of heparan sulfate. The invention further relates to a method for producing recombinant virus particles and to a recombinant virus particle obtainable by the method. The invention further relates to a mammalian packaging cell line deposited under number DSM ACC3355 or DSM ACC3356.

### Background of the invention

Foamy viruses (FV) are a special type of retroviruses that are apparently apathogenic in natural hosts as well as in zoonotically infected humans. Foamy viruses also have an extremely broad tropism. These and other features make foamy viruses an interesting potential viral ferry for the transfer of nucleic acids into cells of a patient in gene therapeutic settings as well as for the transfer of other cargo molecules such as non-foamyviral proteins or nucleoprotein complexes into cells in various applications.

The production of high titer viral vector supernatants of recombinant virus particles that comprise foamy virus envelope glycoprotein (FV Env), for example for nucleic acid transfer applications, requires the use of a special protocol. This protocol employs transient transfection of mammalian packaging cells using cationic polymer-based transfection reagents such as polyethylenimine (PEI). Transfection protocols using standard non-cationic transfection reagents such as calcium phosphate (CaPO) result in 10-fold to 100-fold lower viral vector supernatant titers compared to cationic polymer-based transfection reagents.

For many types of target cells, the transduction efficiency of viral vectors comprising FV Env is superior to vectors comprising other viral glycoproteins such as vesicular stomatitis virus glycoprotein (VSV-G). However, this advantage is not observed for certain target cell types such as myeloid cells and lymphoid cells. The transduction of these cell types is still challenging in general.

Therefore, new tools and methods for producing recombinant virus particles are needed that overcome the current limitations.

### Summary of the invention

In a first aspect, the present invention relates to the use of an engineered mammalian packaging cell line for producing recombinant virus particles, wherein the cell line is engineered to lack cell surface expression of heparan sulfate.

In a second aspect, the present invention relates to a method for producing recombinant virus particles, the method comprising the steps of:
(a) transfecting a plurality of cells of a mammalian packaging cell line engineered to lack cell surface expression of heparan sulfate with
   (i) a transfer vector, and
   (ii) at least one viral packaging plasmid;
   thereby obtaining a plurality of producer cells;
(b) culturing the producer cells in a medium, thereby obtaining the virus particles in the medium; and
(c) separating the medium from the producer cells.

In a third aspect, the present invention relates to a recombinant virus particle obtainable by the method of the invention.

In a further aspect, the present invention relates to a mammalian packaging cell line deposited under number DSM ACC3355 or DSM ACC3356.

### Brief description of the figures

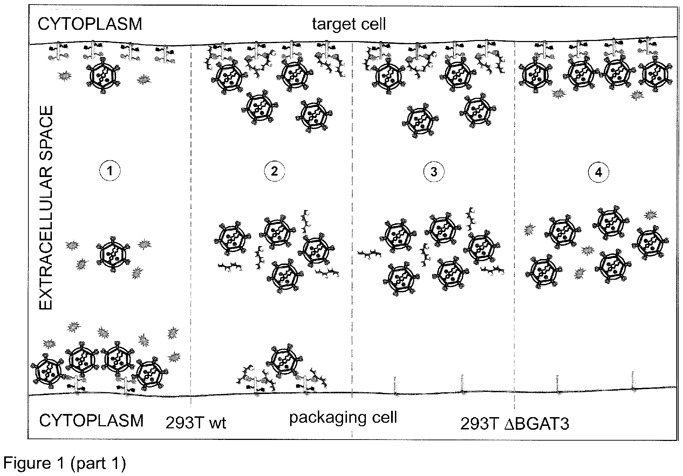

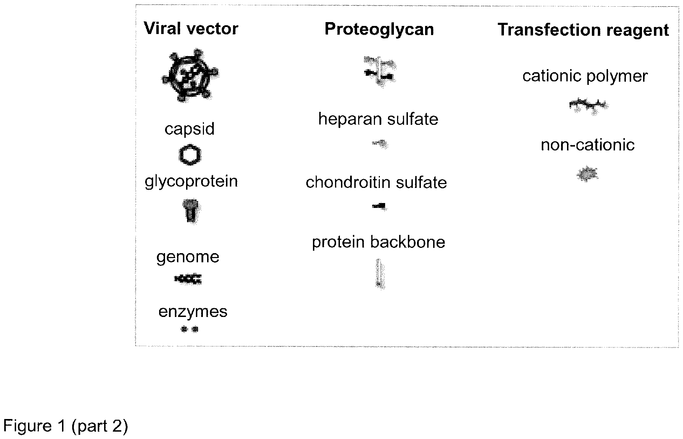

Fig. 1 visualizes some key findings of the inventor in relation to the present invention (293T wt: wild-type HEK293T packaging cells; 293T ΔB3GAT3: HEK293T packaging cells that are engineered to lack an active B3GAT3 protein and that are therefore deficient in cell surface expression of proteoglycans).

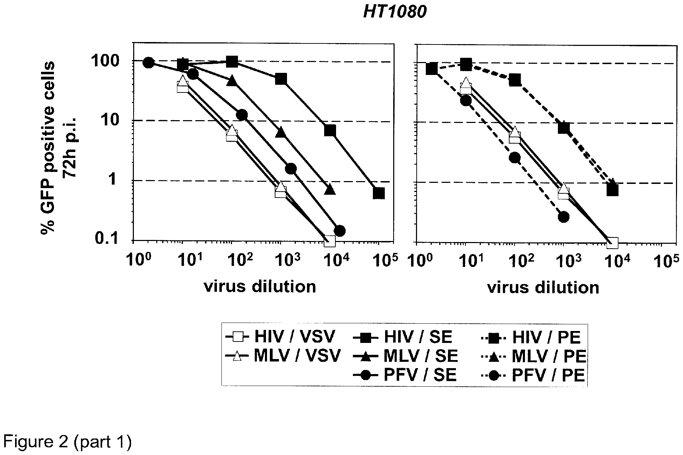

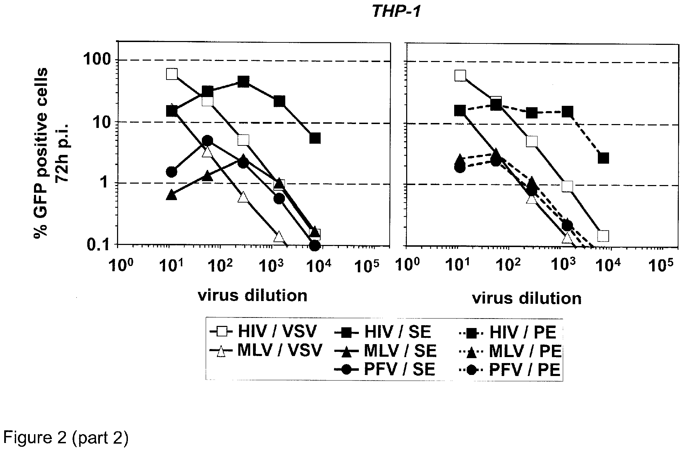

Fig. 2 shows the percentage of GFP expressing cells in samples of HT1080 and THP-1 cells that were incubated with decreasing serial dilutions of GFP encoding retroviral vector particles of human immunodeficiency virus (HIV), murine leukemia virus (MLV) and prototype foamy virus (PFV) origins pseudotyped with different viral glycoproteins as indicated (VSV: vesicular stomatitis virus; SE: macaque simian foamy virus; PE: prototype foamy virus). The percentage of GFP expressing cells was determined seventy-two hours post infection (p.i.) by flow cytometry.

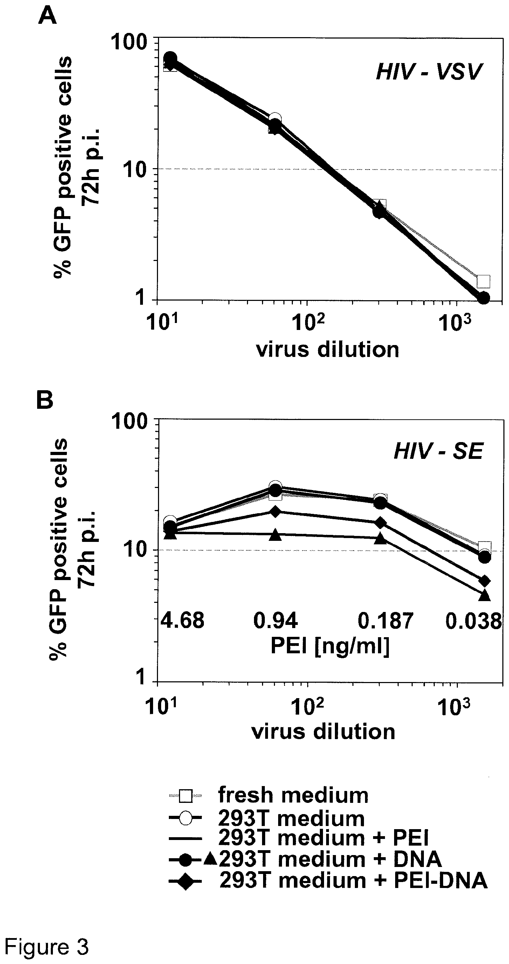

Fig. 3 shows the percentage of GFP expressing cells in samples of THP-1 cells that were incubated with decreasing serial dilutions of GFP encoding HIV vector particles pseudotyped with VSV-G (Fig. 3A) or SFVmac (Fig. 3B) glycoproteins that were mixed at a 1:1 ratio with mock supernatants of different composition as indicated (PEI: polyethylenimine). The percentage of GFP expressing cells was determined seventy-two hours post infection (p.i.) by flow cytometry.

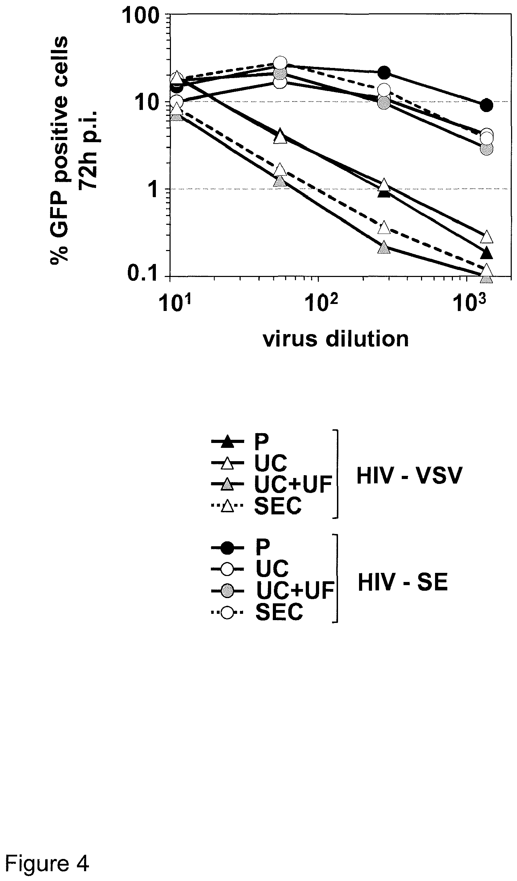

Fig. 4 shows the percentage of GFP expressing cells in samples of THP-1 cells that were incubated with decreasing serial dilutions of GFP encoding HIV vector particles pseudotyped with VSV-G (HIV - VSV) or SFVmac (HIV - SE) glycoproteins that beforehand were further purified as indicated (P: plain 293T supernatants; UC: virus particles pelleted by ultracentrifugation; UC+UF: virus particles pelleted by ultracentrifugation and subsequently concentrated by ultrafiltration; SEC: virus particles purified by size exclusion chromatography). The percentage of GFP expressing cells was determined seventy-two hours post infection (p.i.) by flow cytometry.

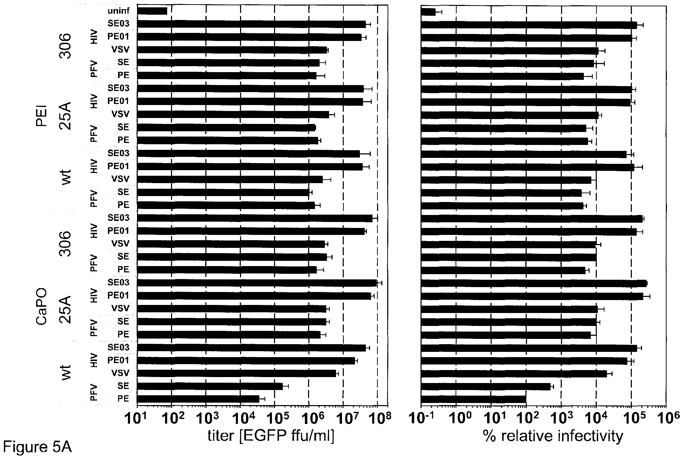

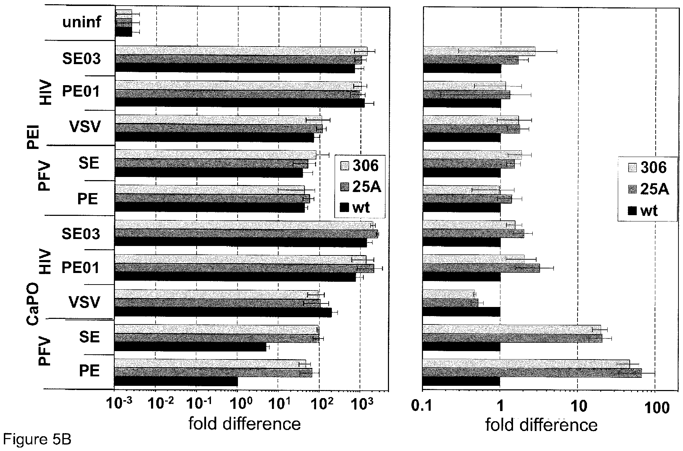

Fig. 5 shows the infectivity of virus particle-harbouring supernatants determined on HT1080 cells using a flow cytometric EGFP transfer assay 72 hours post infection (p.i.). Supernatants were obtained by using different HEK293T packaging cells (wt, 25A, 306) and different transfection techniques (PEI, CaPO). Fig. 5A, left panel, shows the mean values and standard deviations (StdDev; n=3) of absolute viral titers on HT1080 target cells (uninf: uninfected control). Fig. 5A, right panel, shows the infectivity of supernatants relative to PFV vector supernatants containing PFV Env and derived by transient transfection of parental HEK293T (wt) packaging cells using calcium phosphate transfection technique. Fig. 5B, left panel, shows the fold difference of viral titers of individual samples relative to PFV vector supernatants containing PFV Env and derived by transient transfection of parental 293T (wt) packaging cells using calcium phosphate transfection technique (uninf: uninfected control). Fig. 5B, right panel, shows the fold difference of viral titers of the individual samples relative to the type of virus vector derived by transient transfection of parental HEK293T (wt) packaging cells.

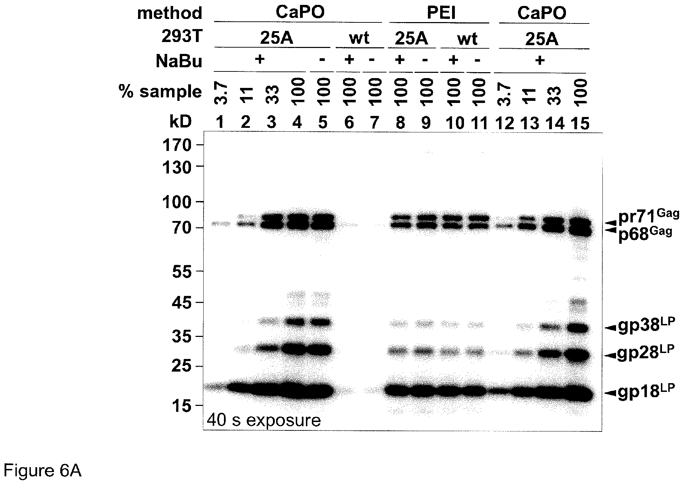

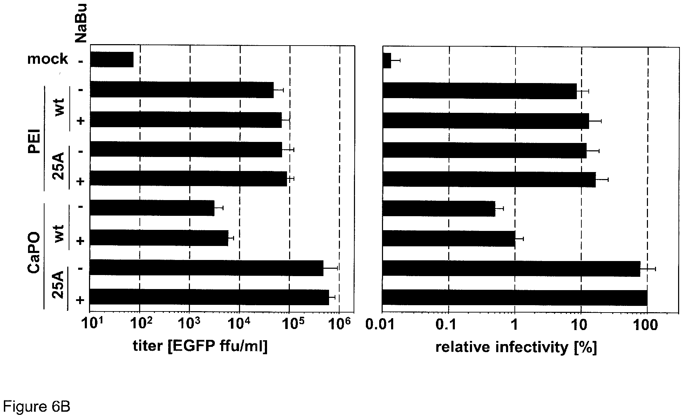

Fig. 6 shows virus particle release and the infectivity of virus particle-harbouring supernatants. Fig. 6A shows Western blot analysis of samples of PFV particles to determine physical virus particle release from different HEK293T packaging cells (wt, 25A) after different transfection techniques (PEI, CaPO) with (+) or without (-) sodium butyrate (NaBu) induction. Fig. 6B shows the infectivity of supernatants determined on HT1080 PLNE cells harbouring a PFV transactivator Tas-dependent EGFP reporter gene expression cassette using a flow cytometric assay at 24 hours post infection. Fig. 6B, left panel, shows mean values and StdDev (n=4) of absolute viral titers of replication-competent viral supernatants on HT1080 PLNE target cells. Fig. 6B, right panel, shows relative infectivity in comparison to virus supernatants produced by CaPO-mediated transient transfection of proteoglycan-deficient HEK293T-25A (25A) packaging cells.

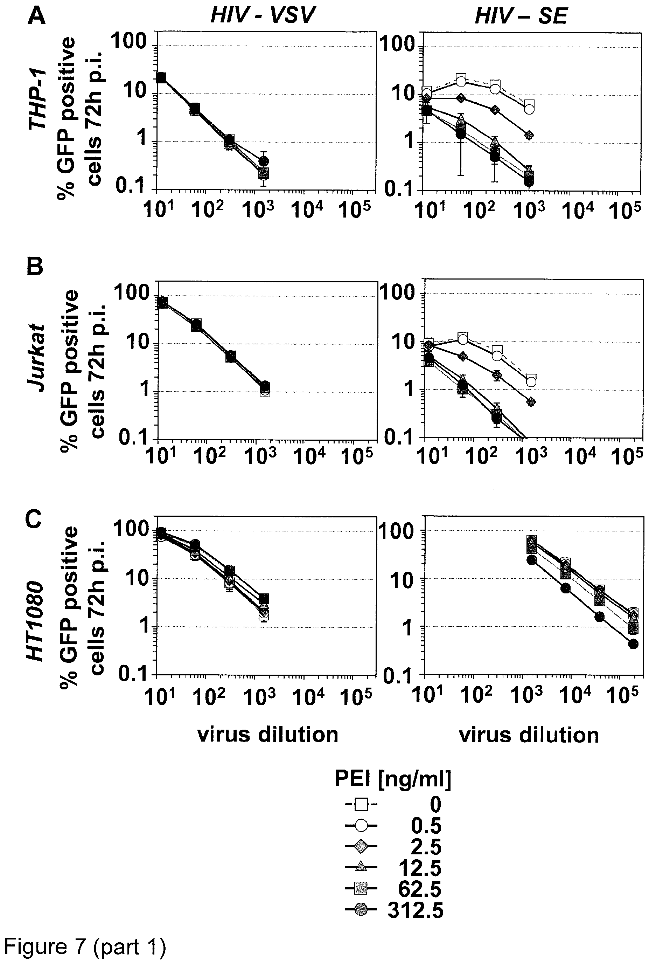

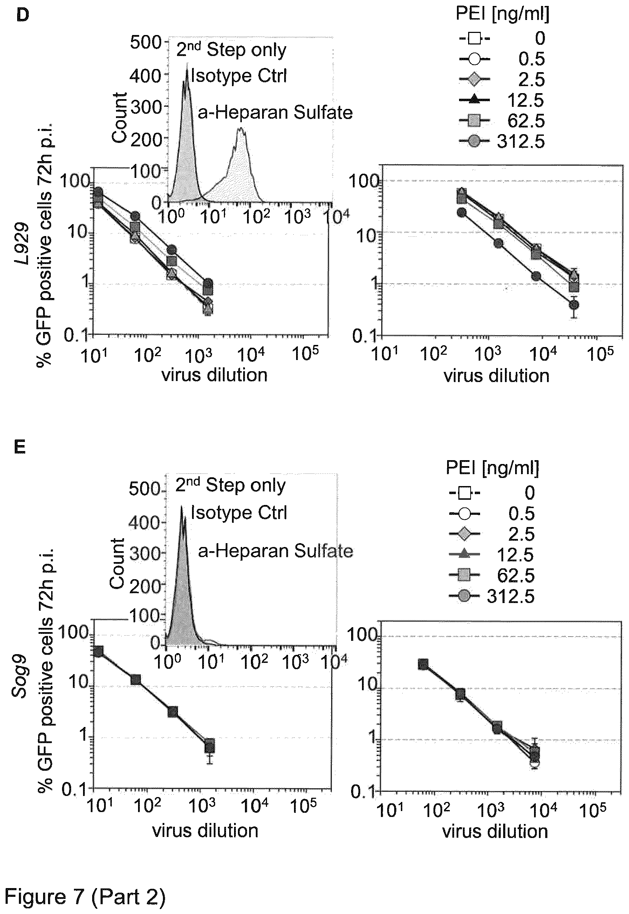

Fig. 7 shows the mean values and StdDev (n=3) of the percentage of GFP expressing cells in samples of different target cells that were incubated with decreasing serial dilutions of GFP encoding HIV vector particles pseudotyped with VSV-G (HIV - VSV) or SFVmac (HIV - SE) glycoproteins that had fixed concentrations of exogenously added PEI as indicated. The HIV vector particles were obtained using parental (wild-type) HEK293T packaging cells using PEI transfection technique. The percentage of GFP expressing cells was determined seventy-two hours post infection (p.i.) by flow cytometry. The insets in the graphs relating to VSV-G infected Mouse L and Sog9 cells (Fig. 7D and 7E) show the FACS staining profiles of heparan sulfate cell surface expression of the respective cell lines (Isotype Ctrl: isotype control).

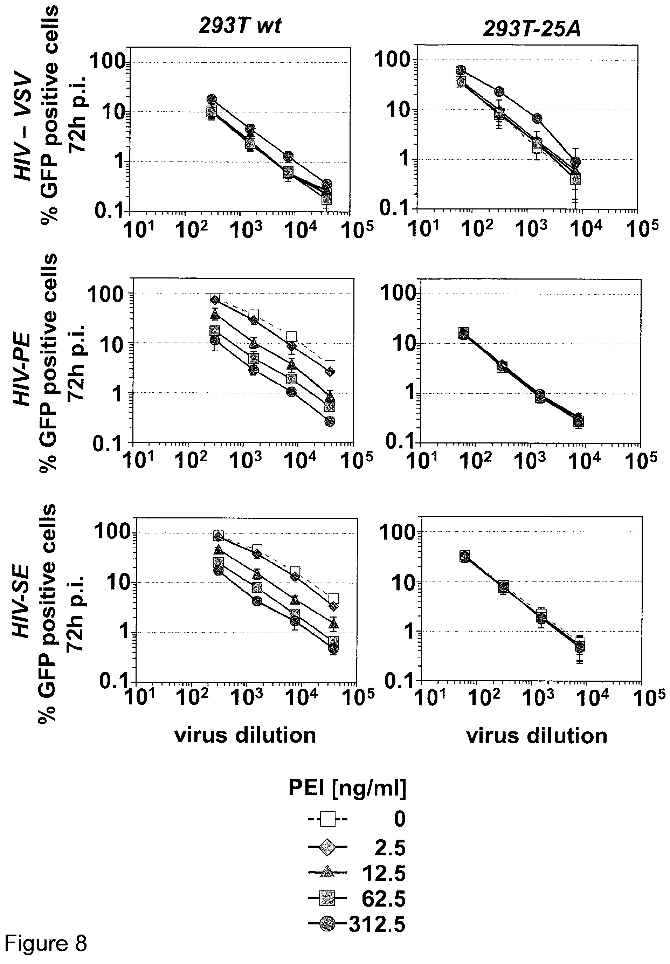

Fig. 8 shows the mean values and StdDev (n=3) of the percentage of GFP expressing cells in samples of parental wild-type HEK293T (293T wt) or proteoglycan-deficient HEK293T ΔB3GAT3 (293T-25A) target cells that were incubated with decreasing serial dilutions of GFP encoding HIV vector particles pseudotyped with VSV-G (HIV - VSV), PFV (HIV - PE) or SFVmac (HIV - SE) glycoproteins that had fixed concentrations of exogenously added PEI as indicated. The HIV vector particles were obtained using proteoglycan-deficient HEK293T ΔB3GAT3 (293T-25A) packaging cell lines using calcium phosphate transfection technique. The percentage of GFP expressing cells was determined seventy-two hours post infection (p.i.) by flow cytometry.

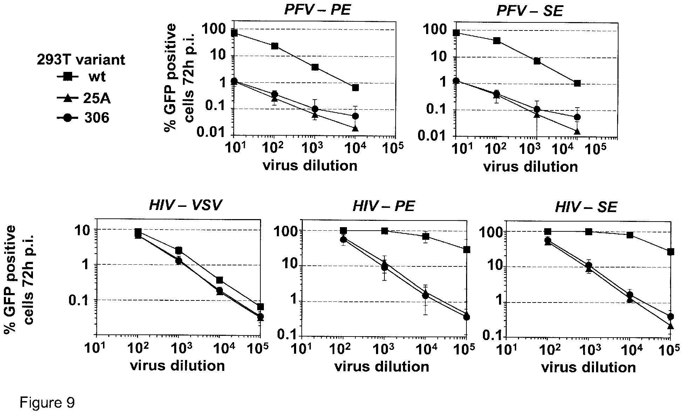

Fig. 9 shows the mean values and StdDev (n=3) of the percentage of GFP expressing cells in samples of parental wild-type HEK293T cells (wt) or proteoglycan-deficient HEK293T ΔB3GAT3 cell variants (25A, 306) that were incubated with decreasing serial dilutions of GFP encoding PFV or HIV vector particles harboring VSV-G (HIV - VSV), PFV (PFV - PE; HIV - PE) or SFVmac (PFV - SE; HIV - SE) glycoproteins as indicated. The PFV and HIV vector particles were obtained using proteoglycan-deficient HEK293T ΔB3GAT3 (293T-25A) packaging cell lines using calcium phosphate transfection technique. The percentage of GFP expressing cells was determined seventy-two hours post infection (p.i.) by flow cytometry.

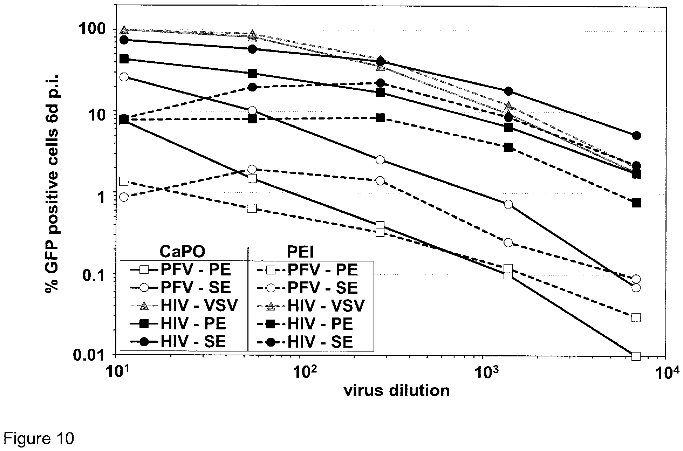

Fig. 10 shows the percentage of GFP expressing cells in samples of THP-1 cells that were incubated with decreasing serial dilutions of different viral vector supernatants as indicated. Data of a representative experiment (n=3) are shown. GFP encoding PFV (PFV) and HIV-1 (HIV) vector supernatants harboring various viral glycoproteins (VSV: vesicular stomatitis virus glycoprotein; PE: PFV envelope glycoprotein; SE: SFVmac glycoprotein) were produced by transient transfection of proteoglycan-deficient HEK293T ΔB3GAT3 (293T-25A) packaging cells using different transfection techniques (PEI, CaPO). The percentage of GFP expressing cells was determined six days post infection (p.i.) by flow cytometry.

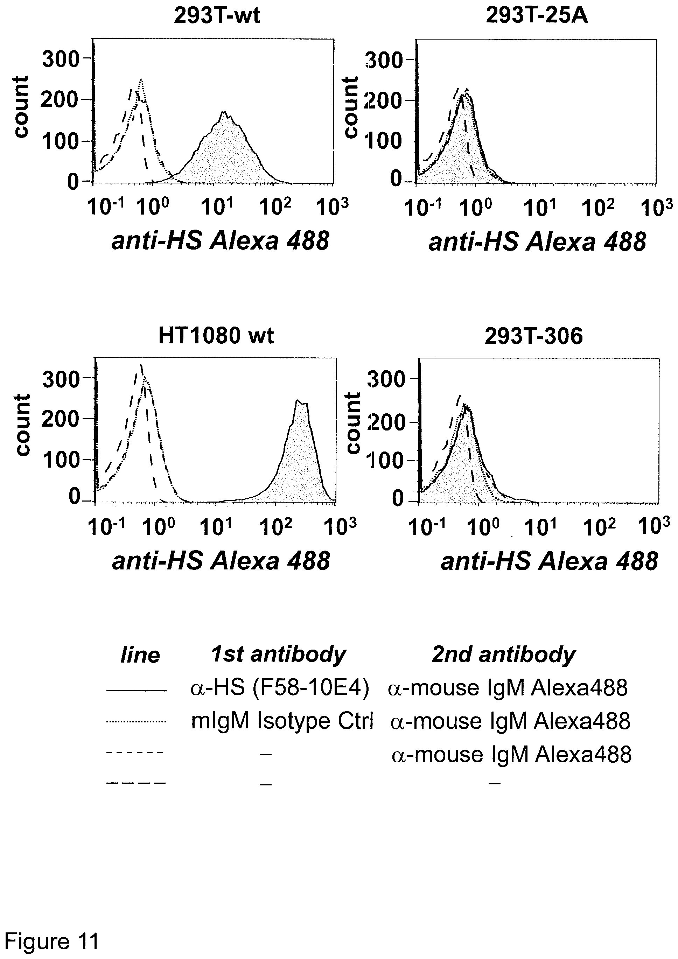

Fig. 11 shows flow cytometry analysis of cell surface heparan sulfate (HS) expression of parental wild-type HEK293T cells (293T-wt), proteoglycan-deficient HEK293T ΔB3GAT3 cell variants (293T-25A, 293T-306) and wild-type HT1080 cells (HT1080 wt). Fig. 11 shows overlay histograms of mouse anti-HS (solid lines, grey area) or mouse IgM K isotype control stainings or further control samples as indicated (dashed lines, white area).

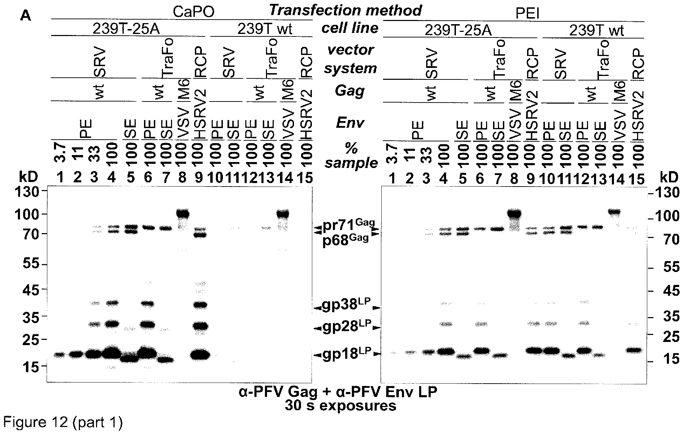

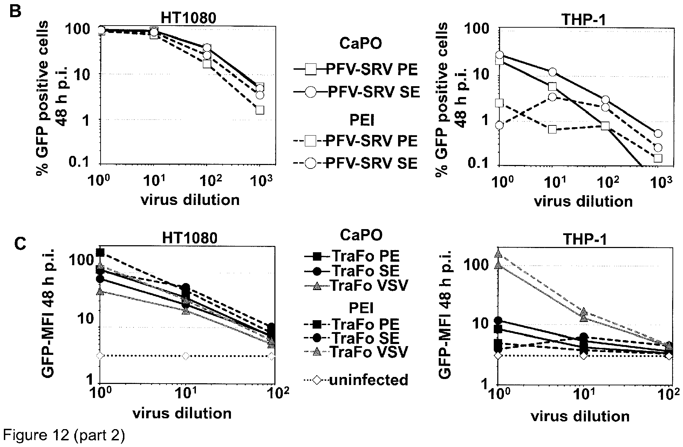

Fig. 12 shows the results of transient transfection of parental 293T (293T wt) or proteoglycan-deficient 293T variant (293T-25A) packaging cell lines with PFV proviral expression constructs for production of replication-competent PFV (RCP), or expression constructs for production of *EGFP* encoding replication-deficient PFV single-round vector particles (SRV), or expression constructs for production of *EGFP* encoding replication-deficient PFV RNA Transfer vector particles (TraFo) harboring PFV Env (PE), SFVmac Env (SE) or VSV-G (VSV) as indicated, using polyethylenimine (PEI) or calcium phosphate (CaPO) transfection methods. Fig. 12A shows Western blot analysis of samples of PFV particles to determine physical particle release. Fig. 12B and 12C show the infectivity of supernatants determined on HT1080-PLNE (HT1080) or THP-1 target cells using a flow cytometric assay at 48 hours post infection (p.i.). Fig. 12B shows the percentage of GFP expressing cells (%-GFP) of HT1080-PLNE or THP-1 cell samples incubated with serial dilutions of the same PFV-SRV supernatants harboring PE or SE Env, produced by CaPO or PEI transfection methods using proteoglycan-deficient 293T-25A packaging cells. Fig. 12C shows the mean fluorescent intensity of the viable cell population in the GFP channel (MFI-GFP) of HT1080-PLNE or THP-1 cell samples incubated with serial dilutions of the same PFV TraFo supernatants harboring PE, SE Env, or VSV-G, produced by CaPO or PEI transfection methods using proteoglycan-deficient 293T-25A packaging cells.

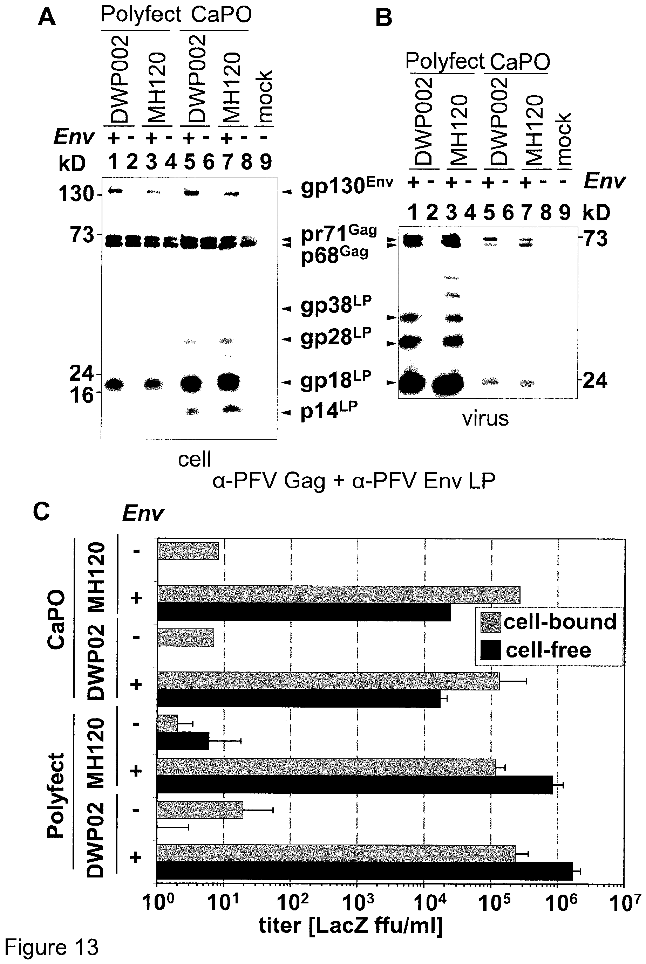

Fig. 13 shows the results of transient transfection of wild-type 293T packaging cells with two variants of a 2-component PFV vector system consisting of a PFV Gag & Pol expressing transfer vector harboring *LacZ* reporter protein expression cassette (DWP02, MH120) and either a PFV Env packaging plasmid (+) or pCDNA3.1 (-). The cells were transfected using Polyfect or calcium phosphate (CaPO) transfection methods as indicated. Fourty-eight hours post transfection cell-free virus supernatants were harvested by sterile filtration of the medium and cell-bound virus supernatants by addition of fresh medium to the transfected packaging cells, a single freeze-thaw cycle and removing cell debris by centrifugation and sterile filtration. Fig. 13A and 13B show Western blot analysis of lysates from packaging cells (cell) transfected with the 2-component PFV vector system expression constructs (Fig. 13A) using Polyfect and CaPO transfection methods as well as PFV particles (virus) to determine cell-free physical particle release (Fig. 13B). Fig. 13C shows the infectivity of cell-free and cell-bound virus supernatants determined on HT1080 target cells using a LacZ gene transfer assay at seventy-two hours post infection. LacZ expressing cells were visualized by histochemical β-galactosidase staining and counting of "blue" foci in individual wells (ffu, focus-forming units). Shown are mean values ± StdDev (n=3).

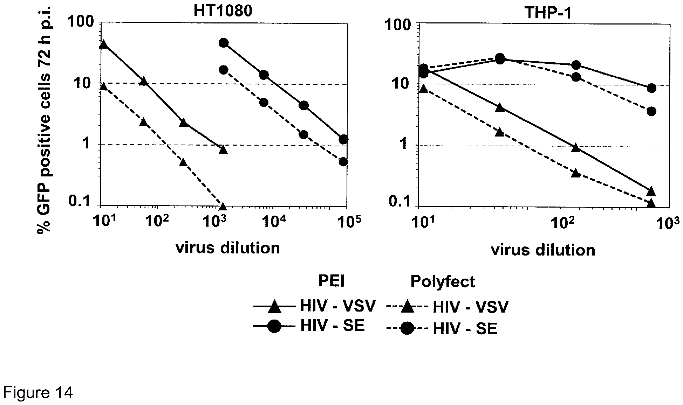

Fig. 14 shows the percentage of GFP expressing cells in samples of HT1080 or THP-1 target cells that were incubated with decreasing serial dilutions of different GFP encoding retroviral vector supernatants as indicated. GFP encoding HIV vector particles pseudotyped with VSV-G (HIV - VSV) or SFVmac (HIV - SE) glycoproteins were produced by transiently transfecting the respective expression plasmids of replication-deficient 3-component HIV-1 vector system into wild-type 293T packaging cells using polyethylenimine (PEI) or Polyfect transfection methods. The percentage of GFP expressing cells was determined seventy-two hours post infection (p.i.) by flow cytometry.

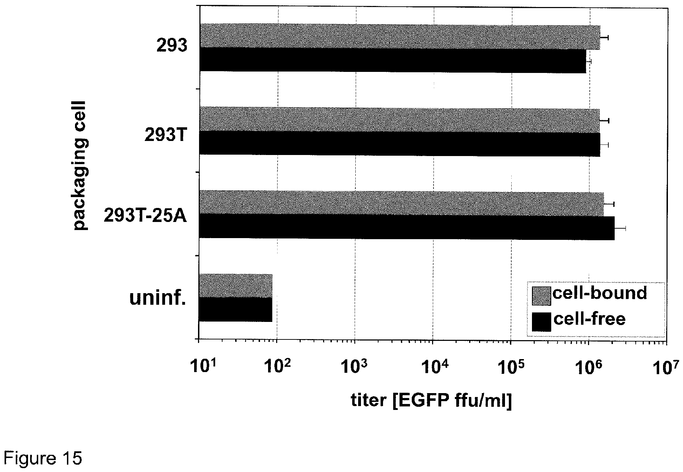

Fig. 15 shows the percentage of GFP expressing cells in samples of HT1080 target cells that were incubated with decreasing serial dilutions of different GFP encoding viral vector supernatants as indicated. GFP encoding recombinant adeno-associated virus (rAAV) vector particles were produced by transient transfection of different packaging cell lines as indicated (293: wild-type HEK293 cell line; 293T: wild-type HEK293T cell line; 293T-25A: B3GAT3-deficient 293T cell line; uninf.: uninfected control). Cell-free rAAV vector particles (cell-free) released from the producer cells were harvested from the cell culture supernatant by low speed centrifugation. Cell-bound rAAV vector particles (cell-bound) retained within the packaging cells and released by freeze-thawing of producer cells in medium were harvested by high speed separation of the lysate and separation of the cellular debris pellet. The percentage of GFP positive cells was determined four days post infection (p.i.) by flow cytometry.

### Detailed description of the invention

In a first aspect, the invention relates to the use of an engineered mammalian packaging cell line for producing recombinant virus particles, wherein the cell line is engineered to lack cell surface expression of heparan sulfate.

The term "packaging cell line" as used herein refers to a cell line that is suitable for viral packaging, i.e. for the *in vitro* production of recombinant virus particles. This implies that the cells of the packaging cell line can be transfected with a transfer vector and one or more viral packaging plasmids to produce and release recombinant virus particles. Once transfected, the packaging cells are also referred to as "producer cells". The virus particles are released into the medium in which the cells are cultivated. Unless indicated otherwise, the term "supernatant" as used herein refers to the medium in which the recombinant virus particles are present due to their release from the producer cells. The supernatant is also referred to as viral supernatant or viral vector supernatant herein.

Where indicated, the term "supernatant" also refers to medium in which recombinant virus particles are present due to freeze-thawing of producer cells and subsequent removal of cell debris by low-speed centrifugation and/or sterile filtration (using, for example, a 0.45 µm filter). Freeze-thawing of producer cells is used to release cell-bound virus particles.

The term "engineered cell line" as used herein refers to a modified cell line. The engineered cell line is derived from a parental cell line, in which one or more modifications have been introduced. Due to the modification introduced, the engineered cell line differs from the parental cell line. The cell line may, for example, be engineered (modified) to have an altered protein expression profile compared to the parental cell line. The expression of a given protein may be increased or reduced for example by inserting or deleting the gene, which encodes the protein, or by activating or inactivating the gene in the cells' genome, respectively. The expression of a given protein may also be altered on the epigenetic level or on the mRNA level, i.e. by interfering with the transcription of the gene encoding the protein or by interfering with the translation of the corresponding mRNA, respectively. The engineered cell line may, for example, be engineered to have an altered protein sorting pathway compared to the parental cell line, which may result in an altered composition of the engineered cell line's cell surface molecules.

The engineered cell line preferably is a genetically engineered cell line, i.e. a cell line in which the one or more modifications have been artificially introduced on the gene level (i.e. in the genome) of the cell line. In this case, the one or more modifications are stably introduced into the cells and passed on to subsequent generations of the cells. Examples of genetically engineered cell lines comprise cell lines in which one or more genes are inserted, deleted, or activated or inactivated on the gene level. Activation or inactivation of a gene on the gene level may be achieved by introducing mutations into the gene, for example by using a targeting endonuclease mediated genome editing method. The targeting endonuclease may be, for example, an RNA-guided Cas (CRISPR-associated) endonuclease.

The engineered cell line may alternatively be a cell line in which the one or more modifications have been introduced on the gene expression level or on the protein level of the cell line.

The inventor found that a mammalian packaging cell line that is engineered to lack cell surface expression of heparan sulfate is particularly useful for producing recombinant virus particles.

Heparan sulfate is a polysaccharide that belongs to the family of glycosaminoglycans (GAGs). GAGs (also called mucopolysaccharides) are long unbranched polysaccharide chains characterized by a backbone of repeating disaccharide units and inserted aminosugars and uronic acids. The specific types of those additional molecules result in different types of GAGs. For example, chondroitin sulfate incorporates N-acetylgalactosamine and glucuronic acid, while heparan sulfate comprises N-acetylglucosamine and glucuronic acid. GAGs are classified into four groups: (i) heparin/heparan sulfate GAGs, (ii) chondroitin sulfate/dermatan sulfate GAGs, (iii) keratan sulfates and (iv) hyaluronic acid.

At the cell surface, heparan sulfate is present in the form of heparan sulfate proteoglycan. Proteoglycans are cell surface molecules that have a core cell surface protein with one or more GAG chains covalently attached to it via certain linker regions. A heparan sulfate proteoglycan (HSPG) generally comprises several heparan sulfate (HS) chains, such as two or three HS chains, which are attached to the cell surface protein. In this form, heparan sulfate binds to a variety of protein ligands and regulates a wide range of biological activities.

The term "proteoglycan" as used herein comprises HSPGs as well as chondroitin sulfate/dermatan sulfate proteoglycans. Like heparan sulfate, chondroitin sulfate and dermatan sulfate are synthesized in the Golgi apparatus, where protein cores made in the rough endoplasmic reticulum are posttranslationally modified with O-linked glycosylations by glycosyltransferases forming the proteoglycans. The term "proteoglycan" thus refers to a cell surface molecule that comprises (i) a protein, and (ii) at least one heparan sulfate chain, chondroitin sulfate chain or dermatan sulfate chain attached to the protein.

The inventor observed that when using standard packaging cells for producing recombinant virus particles, the production of high titer viral vector supernatants of FV Env-comprising virus particles is strongly dependent on the type of transfection reagent used. Transfection protocols employing standard non-cationic transfection reagents such as calcium phosphate, which is frequently used for producing vesicular stomatitis virus glycoprotein (VSV-G) pseudotypes of lentiviral vector particles, only yield very poor titers of FV Env-comprising retroviral vector particles. For example, transfection by calcium phosphate coprecipitation results in 10- to 100-fold lower viral vector supernatant titers of FV Env-comprising virus particles compared to transfection by polyethylenimine or Polyfect, which are commonly used cationic polymer-based transfection reagents.

Polyfect is a transfection reagent that is commercially available from the company Qiagen. It consists of dendrimer molecules of a defined spherical architecture with branches radiating from a central core. The branches terminate at charged amino groups, which can interact with negatively charged phosphate groups of nucleic acids.

The invention is based on the finding that heparan sulfate is the major cellular factor influencing the virus titer of viral vector supernatants when comparing the use of cationic polymer-based or non-cationic transfection reagents for the production of recombinant virus particles. It was previously shown that heparan sulfate plays a role in FV Env-mediated infection of target cells. It was found that various FV species, like many other viruses, use heparan sulfate as an attachment factor in a FV Env-dependent manner. When present on target cells, heparan sulfate enhances viral infectivity of FV Env-comprising virus particles by promoting virus attachment. However, heparan sulfate is not an essential entry factor for FV Env-mediated fusion of viral and cellular lipid membranes as cells deficient in cell surface expression of heparan sulfate are not resistant towards FV Env-mediated infection.

Surprisingly, the inventor found that heparan sulfate also plays a role in the release of recombinant virus particles into the cell culture supernatants of packaging cells. The inventor found that cationic polymer-based transfection reagents such as polyethylenimine and Polyfect neutralize free heparan sulfate binding sites on the cell surface of standard packaging cells, thereby enabling a more efficient release of FV Env-comprising virus particles into the cell culture supernatants and/or preventing the adsorption of released virus particles to heparan sulfate on the cell surface of the packaging cells. This allows the production of high titer, cell-free, FV Env-comprising viral vector supernatants when cationic polymer-based transfection reagents are used (Fig. 1, Panel 2). In contrast, when non-cationic transfection reagents such as calcium phosphate are used, the heparan sulfate binding sites on the cell surface of the standard packaging cells are not neutralized and therefore, FV Env-comprising virus particles are not efficiently released from the packaging cells and/or are readily adsorbed to the cell surface of the packaging cells. This results in only low amounts of virus particles in cell-free viral vector supernatants when non-cationic transfection reagents are used (Fig. 1, Panel 1). The low titers of the virus particles contribute to poor target cell transduction.

These findings were confirmed in an ultrastructural analysis of the morphogenesis of FV Env-comprising viruses using electron microscopy of transfected HEK293T packaging cells. The analysis revealed marked differences in the amounts of detectable budding viral particle structures depending on the transfection method used. In samples of calcium phosphate transfected packaging cells, numerous budding viral particle structures comprising characteristic FV glycoprotein spike structures at the plasma membrane and intracellular compartments were detectable. This confirms that the virus particles are not efficiently released from the packaging cells since the packaging cells retain the virus particles. In contrast, in samples of polyethylenimine transfected packaging cells, the number of budding viral particle structures was strongly reduced and hard to detect at all. This is in line with the efficient release of the virus particles from the packaging cells.

Based on the newly discovered role of heparan sulfate, the inventor generated and tested a packaging cell line for producing recombinant virus particles that is engineered to lack cell surface expression of heparan sulfate. The inventor found that by using a packaging cell line that is engineered to lack cell surface expression of heparan sulfate, FV Env-comprising virus particles can no longer be retained and/or adsorbed to the cell surface of the engineered packaging cells in case non-cationic transfection reagents are used for virus production. The use of a packaging cell line that is engineered to lack cell surface expression of heparan sulfate thus facilitates the production of viral supernatants with a high titer of recombinant virus particles even when non-cationic transfection reagents are used. Surprisingly, the inventor found that FV Env-comprising virus vector titers obtained by transfection of packaging cells by calcium phosphate coprecipitation (i.e. by using a non-cationic transfection reagent) are up to 100-fold higher when using a packaging cell line that is engineered to lack cell surface expression of heparan sulfate compared to using the corresponding parental packaging cell line. In line with this finding, the inventor also found a significantly increased physical virus particle release from packaging cells transfected by calcium phosphate coprecipitation when using a packaging cell line that is engineered to lack cell surface expression of heparan sulfate compared to using the corresponding parental packaging cell line.

The inventor also analysed the amount of cell-bound FV Env-comprising virus particles that are present in packaging cells transfected by calcium phosphate coprecipitation when using a packaging cell line that is engineered to lack cell surface expression of heparan sulfate compared to using the corresponding parental packaging cell line. The inventor found that cell-bound virus titers are similar, indicating that the amount of virus particles produced by the packaging cells is so high that the increased virus particle release from the engineered packaging cells does not lead to a considerable reduction of the amount of cell-bound virus particles present in the cells.

Transfection of the engineered packaging cell line or the corresponding parental packaging cell line by cationic polymer-based transfection reagents such as polyethylenimine results in similar FV Env-comprising virus vector titers.

The invention thus facilitates a production system for viral vector supernatants that is independent of cationic polymer-based transfection reagents. At the same time, the production system still allows production of viral vector supernatants with similar or higher titers as obtained by current state of the art systems such as those using unmodified packaging cells and cationic polymer-based transfection reagents.

When comparing the use of cationic polymer-based or non-cationic transfection reagents for the production of recombinant virus particles in a packaging cell line that is engineered to lack cell surface expression of heparan sulfate, the inventor found that FV Env-comprising virus vector titers obtained by using a non-cationic transfection reagent are up to 10-fold higher compared to virus titers obtained by using cationic polymer-based transfection reagents.

Even though FV Env-comprising virus particles were used in the studies underlying the present invention, the present invention is not limited to FV Env-comprising virus particles. The findings of the inventor likewise apply to all recombinant virus particles that interact with cell surface heparan sulfate. Heparan sulfate is known to serve as a cellular entry receptor or as an attachment factor for many viruses on the cell surface of their target cells.

The use of non-cationic transfection reagents, which is facilitated by the engineered packaging cell line, results in the absence of remnants of cationic polymer-based transfection reagents in the viral vector supernatants. This is advantageous since the inventor found that remnants of cationic polymer-based transfection reagents in viral vector supernatants act as inhibitory factor that reduces viral vector infectivity by blocking heparan sulfate binding sites on the cell surface of target cells, thereby diminishing virus particle attachment to the target cells (Fig. 1, Panels 2 and 3). In particular, remnants of cationic polymer-based transfection reagents in viral vector supernatants were found to reduce viral vector infectivity in certain types of target cells such as myeloid and lymphoid target cells, in particular at high virus concentrations of FV Env-comprising virus particles. In contrast, for FV Env-comprising viral vector supernatants obtained by non-cationic transfection reagents, a dose-dependent increase in viral vector infectivity in myeloid target cells was observed with the use of increasing amounts of viral vector supernatant.

The use of a packaging cell line that is engineered to lack cell surface expression of heparan sulfate thus facilitates the production of high titer recombinant virus particles with superior transduction efficiency in great variety of target cells, in particular in myeloid and lymphoid cells (Fig. 1, Panel 4). It thereby facilitates efficient virus-mediated gene transfer into a great variety of target cells.

The use of a packaging cell line that is engineered to lack cell surface expression of heparan sulfate will be useful for the production of any type of transfer vehicle (such as, for example, gene transfer vehicle) that comprises natural or artificially introduced binding sites for heparan sulfate or that is influenced by interactions with heparan sulfate, such as, for example, adeno-associated virus.

The absence of heparan sulfate expression on the cell surface of packaging cells can be easily confirmed by routine methods known in the art such as flow cytometry. For flow cytometry, cells are generally stained with an antibody that is specific for a molecule of interest such as, for example, heparan sulfate. The antibody may be fluorescently labelled itself or it may be detected by staining with a fluorescently labelled secondary antibody directed against the first (primary) antibody. The cells are then analysed for the presence or absence of the fluorescent label using a flow cytometer.

The term "transfection of cells" as used herein refers to the process of introducing one or more foreign nucleic acids into the cells using physical or chemical methods. Several transfection techniques are known, for example electroporation, cationic liposome-mediated transfection, calcium phosphate coprecipitation, or methods using cationic polymers such as DEAE-dextran or polyethylenimine. For the transfection of packaging cells to produce recombinant virus particles, calcium phosphate coprecipitation or cationic polymer-based transfection reagents, in particular polyethylenimine, are commonly used to date.

In contrast, the term "transduction of cells" as used herein refers to the process of transferring one or more nucleic acids or other cargo molecules such as heterologous viral proteins, non-viral proteins, nucleoprotein complexes, sugars or drugs into the cells using a DNA virus or an RNA virus. The virus is typically a recombinant virus that has been produced by a suitable packaging cell. An important application of the transduction of cells by recombinant virus particles is the virus-mediated delivery of one or more genes into target cells, for example into target cells of a patient in gene therapy.

The term "transduction efficiency" refers to the proportion or percentage of target cells that are successfully transduced by virus particles after transduction of the cells. Transduction efficiency is also referred to as infectivity, viral infectivity or viral vector infectivity herein.

The term "target cell" as used herein refers to a cell that is to be transduced (infected) by a recombinant virus particle.

The term "gene therapy" as used herein refers to the therapeutic delivery of a nucleic acid into cells of a patient to treat a disease.

In a preferred embodiment, the cell surface expression of heparan sulfate is lacking due to the cell line being engineered not to have a functional heparan sulfate proteoglycan (HSPG) synthesis pathway. In other words, the cell line is engineered to be a HSPG deficient cell line. As mentioned above, at the cell surface, heparan sulfate is present in the form of HSPG. The HSPG (bio-)synthesis pathway of the packaging cell line may be non-functional, for example, by inactivation of one or more essential enzymes of the pathway. Accordingly, the cell line may be engineered to lack one or more active enzymes of the HSPG synthesis pathway. The one or more enzymes are preferably selected from the group consisting of beta-1,3-glucuronyltransferase 3 (B3GAT3), xylosyltransferase 1 (XYLT1), xylosyltransferase 2 (XYLT2), beta-1,4-galactosyltransferase 7 (B4GALT7), beta-1,3-galactosyltransferase 6 (B3GALT6), β3-GlcA (glucuronic acid) transferase, exostosin glycosyltransferase 1 (EXT1), exostosin glycosyltransferase 2 (EXT2), glucuronic acid epimerase (GLCE), exostosin-like glycosyltransferase 1 (EXTL1), exostosin-like glycosyltransferase 2 (EXTL2), exostosin-like glycosyltransferase 3 (EXTL3), N-deacetylase and N-sulfotransferase 1 (NDST1), N-deacetylase and N-sulfotransferase 2 (NDST2), N-deacetylase and N-sulfotransferase 3 (NDST3), N-deacetylase and N-sulfotransferase 4 (NDST4), heparan sulfate 2-O-sulfotransferase 1 (HS2ST1), heparan sulfate 6-O-sulfotransferase 1 (HS6ST1), heparan sulfate 6-O-sulfotransferase 2 (HS6ST2), heparan sulfate 6-O-sulfotransferase 3 (HS6ST3), and heparan sulfate-glucosamine 3-sulfotransferase 1 (HS3ST1), heparan sulfate-glucosamine 3-sulfotransferase 2 (HS3ST2), heparan sulfate-glucosamine 3-sulfotransferase 3A1 (HS3ST3A1), heparan sulfate-glucosamine 3-sulfotransferase 3B1 (HS3ST3B1), heparan sulfate-glucosamine 3-sulfotransferase 4 (HS3ST4), heparan sulfate-glucosamine 3-sulfotransferase 5 (HS3ST5), heparan sulfate-glucosamine 3-sulfotransferase 6 (HS3ST6), sulfatase 1 (SULF1), and sulfatase 2 (SULF2). These enzymes are examples of enzymes that are necessary for a functional HSPG synthesis pathway. The lack of one or more of these enzymes results in a defective HSPG synthesis pathway and thus in the absence of cell surface expression of HSPGs.

In a preferred embodiment, the one or more enzymes are selected from the group consisting of exostosin glycosyltransferase 1 (EXT1), exostosin glycosyltransferase 2 (EXT2), glucuronic acid epimerase (GLCE), exostosin-like glycosyltransferase 1 (EXTL1), exostosin-like glycosyltransferase 2 (EXTL2), exostosin-like glycosyltransferase 3 (EXTL3), N-deacetylase and N-sulfotransferase 1 (NDST1), N-deacetylase and N-sulfotransferase 2 (NDST2), N-deacetylase and N-sulfotransferase 3 (NDST3), N-deacetylase and N-sulfotransferase 4 (NDST4), heparan sulfate 2-O-sulfotransferase 1 (HS2ST1), heparan sulfate 6-O-sulfotransferase 1 (HS6ST1), heparan sulfate 6-O-sulfotransferase 2 (HS6ST2), heparan sulfate 6-O-sulfotransferase 3 (HS6ST3), heparan sulfate-glucosamine 3-sulfotransferase 1 (HS3ST1), heparan sulfate-glucosamine 3-sulfotransferase 2 (HS3ST2), heparan sulfate-glucosamine 3-sulfotransferase 3A1 (HS3ST3A1), heparan sulfate-glucosamine 3-sulfotransferase 3B1 (HS3ST3B1), heparan sulfate-glucosamine 3-sulfotransferase 4 (HS3ST4), heparan sulfate-glucosamine 3-sulfotransferase 5 (HS3ST5), heparan sulfate-glucosamine 3-sulfotransferase 6 (HS3ST6), sulfatase 1 (SULF1), and sulfatase 2 (SULF2), more preferred from the group consisting of exostosin glycosyltransferase 1 (EXT1), exostosin glycosyltransferase 2 (EXT2), glucuronic acid epimerase (GLCE), exostosin-like glycosyltransferase 1 (EXTL1), exostosin-like glycosyltransferase 2 (EXTL2) and exostosin-like glycosyltransferase 3 (EXTL3). These enzymes are specific for the HSPG synthesis pathway.

An enzyme of the HSPG synthesis pathway may be inactivated by inactivation of the gene encoding the enzyme, preferably by CRISPR (clustered regularly interspaced short palindromic repeats)/Cas (CRISPR-associated) mediated inactivation of the gene, so that the cells do not express the respective enzyme. CRISPR/Cas mediated genome editing is a preferred technique for genetically inactivating a gene. By introducing a specifically engineered guideRNA (gRNA) or single-guided RNA (sgRNA) into the CRISPR/Cas system, any region of a genome can be targeted in a very precise way. In particular, the CRISPR/Cas9 system (Jinek, M. et al. 2012) can easily be used as a genetic engineering tool.

An enzyme of the HSPG synthesis pathway may alternatively be inactivated by other means. For example, the enzyme may be inactivated on the protein level by adding a suitable enzyme inhibitor to the packaging cells. The use of an enzyme inhibitor facilitates a time-controllable transient inactivation of the enzyme. This may be advantageous in case the active version of the enzyme is required or beneficial for other cellular processes.

As another example, the enzyme may be inactivated on the gene expression level by silencing the expression of the gene encoding the enzyme. This can be achieved, for example, by means of RNA interference using, for example, siRNA molecules or microRNA molecules.

In another embodiment, the cell surface expression of heparan sulfate is lacking due to the cell line being engineered not to have a functional protein sorting pathway for sorting heparan sulfate-harbouring proteoglycans to the cell surface.

In a preferred embodiment, the cell line is engineered to further lack cell surface expression of chondroitin sulfate and dermatan sulfate. As mentioned above, like heparan sulfate, chondroitin sulfate and dermatan sulfate are present at the cell surface in the form of proteoglycans. Thus, the cell line that is engineered to further lack cell surface expression of chondroitin sulfate and dermatan sulfate is a proteoglycan deficient cell line. In other words, the cell line that is engineered to further lack cell surface expression of chondroitin sulfate and dermatan sulfate is a cell line that is engineered to lack cell surface expression of proteoglycans.

Depending on the envelope protein of recombinant virus particles, chondroitin sulfate and dermatan sulfate may also play a role in the release of the recombinant virus particles into the cell culture supernatants of packaging cells as was found for heparan sulfate and FV Env-comprising virus particles. Therefore, a cell line that is engineered to lack cell surface expression of heparan sulfate, chondroitin sulfate and dermatan sulfate is also useful for producing recombinant virus particles and will be particularly useful for producing viruses that primarily interact with cell surface chondroitin sulfate and/or dermatan sulfate.

In a preferred embodiment, the cell surface expression of heparan sulfate, chondroitin sulfate and dermatan sulfate is lacking due to the cell line being engineered not to have a functional proteoglycan synthesis pathway. In other words, the cell line is engineered to be a proteoglycan deficient cell line. The proteoglycan (bio-)synthesis pathway of the packaging cell line may be non-functional, for example, by inactivation of one or more essential enzymes of the pathway. Accordingly, the cell line may be engineered to lack one or more active enzymes of the proteoglycan synthesis pathway. The one or more enzymes are preferably selected from the group consisting of beta-1,3-glucuronyltransferase 3 (*B3GAT3,* GlcAT-I), xylosyltransferase 1 (*XYLT1,* XylT), xylosyltransferase 2 (*XYLT2,* XylT), beta-1,4-galactosyltransferase 7 (*B4GALT7,* GalT-I), and beta-1,3-galactosyltransferase 6 (*B3GALT6,* GalT-II). These enzymes are involved in the biosynthesis of the tetrasaccharide linkage region which links a heparan sulfate, chondroitin sulfate or dermatan sulfate polysaccharide chain to a proteoglycan core protein in a proteoglycan.

An enzyme of the proteoglycan synthesis pathway may be inactivated by inactivation of the gene encoding the enzyme, preferably by CRISPR/Cas mediated inactivation of the gene, so that the cells do not express the respective enzyme. An enzyme of the proteoglycan synthesis pathway may alternatively be inactivated, for example, on the protein level by adding a suitable enzyme inhibitor to the packaging cells.

In another embodiment, the cell surface expression of heparan sulfate, chondroitin sulfate and dermatan sulfate is lacking due to the cell line being engineered not to have a functional protein sorting pathway for sorting proteoglycans to the cell surface.

In a preferred embodiment, the cell line is engineered to lack an active beta-1,3-glucuronyltransferase 3 (B3GAT3) protein. The B3GAT3 protein is a key enzyme of the proteoglycan synthesis pathway. It is also referred to as galactosylgalactosylxylosylprotein 3-beta-glucuronosyltransferase 3. Further alternative names comprise Glucuronosyltransferase I (GlcAT-I, GLCATI) and UDP-GlcUA:Gal beta-1,3-Gal-R glucuronyltransferase (GlcUAT-I). B3GAT3 is involved in the biosynthesis of the GAG polysaccharide chain tetrasaccharide linkage region on the proteoglycan core protein of heparan sulfate proteoglycans, chondroitin sulfate proteoglycans and dermatan sulfate proteoglycans. More specifically, the B3GAT3 protein transfers a glucuronic acid moiety from the uridine diphosphate-glucuronic acid (UDP-GlcUA) to the common linkage region trisaccharide Gal-beta-1,3-Gal-beta-1,4-Xyl covalently bound to a serine residue at the glycosaminylglycan attachment site of proteoglycans. Therefore, the knockout or inactivation of B3GAT3 impairs the cell surface expression of all sorts of proteoglycans, since heparan sulfate chains, chondroitin sulfate chains and dermatan sulfate chains are not being synthesized on the core protein any more. Accordingly, cells or cell lines that are deficient in an active (functional) B3GAT3 protein lack any type of proteoglycan. Accordingly, the cells are devoid of proteoglycans on their cell surface, i.e. they lack cell surface expression of proteoglycans. Accordingly, the cells lack cell surface expression of heparan sulfate, chondroitin sulfate and dermatan sulfate.

The cell line may be engineered to lack an active B3GAT3 protein via different strategies. For example, the cell line may be genetically engineered to have a deletion of the *B3GAT3* gene encoding the B3GAT3 protein or to have an inactivated version of the gene. As another example, the cell line may be engineered to exhibit silencing of gene expression of the *B3GAT3* gene, for example by means of RNA interference using, for example, siRNA molecules. As another example, the cell line may be engineered to have an inactivated B3GAT3 protein on the protein level, for example by adding inhibitors of the B3GAT3 enzyme to the packaging cells.

In a preferred embodiment, the active B3GAT3 protein is lacking due to the cell line being genetically engineered to lack expression of the B3GAT3 protein. Genetic engineering facilitates a stable modification of the cell line that is passed on to subsequent generations of the cells. The cell line may be genetically engineered to lack expression of the B3GAT3 protein by genetically inactivating the *B3GAT3* gene encoding the B3GAT3 protein, for example by CRISPR/Cas mediated genome editing.

The inventor generated modified clonal HEK293T packaging cells with a defective proteoglycan synthesis pathway by CRISPR/Cas9 mediated inactivation of the *B3GAT3* gene. The inventor isolated single cell clones of the B3GAT3 deficient HEK293T cells and confirmed by flow cytometry analysis that the cells did not show cell surface expression of heparan sulfate. For flow cytometry analysis, cells were stained with a heparan sulfate-specific mouse monoclonal antibody (primary antibody) and a fluorescently labelled anti-mouse antibody as secondary antibody.

In a preferred embodiment, the cell line is derived from a cell line selected from the group consisting of human embryonic kidney 293T (HEK293T) cell line, human embryonic kidney 293 (HEK293) cell line, HeLa cell line, D-17 cell line, HT1080 cell line, TE671 cell line, MV-1-Lu cell line and HOS cell line. These cell lines are known to serve as suitable mammalian packaging cell lines for producing particulate structures such as recombinant virus particles. The corresponding wild-type (parental) cell lines express heparan sulfate on their cell surface.

The HEK293T cell line is a human cell line derived from the HEK293 cell line by modifying the HEK293 cell line to express the SV40 large T antigen to allow improved replication and expression of transfected plasmids containing the SV40 origin of replication. The wild-type HEK293T cell line is a very commonly used packaging cell line for producing recombinant virus particles, in particular retrovirus particles.

HEK293 cells can be used as packaging cells for, for example, recombinant adenovirus (AV) and adeno-associated virus (AAV) particles.

HeLa cells are human epithelial cells derived from cervical cancer. HeLa cells can be used as packaging cells for, for example, recombinant AAV particles.

The D-17 cell line is a canine osteosarcoma cell line. D-17 cells can be used as packaging cells for, for example, recombinant retrovirus particles.

The HT1080 cell line is a human fibroblast cell line. HT1080 cells can be used as packaging cells for, for example, recombinant retroviruses such as murine leukemia viruses (MLV). Wild-type HT1080 cells express great amounts of heparan sulfate on their cell surface.

The TE671 cell line is a human cancer cell line. The MV-1-Lu (also referred to as Mv1Lu) cell line is a mink lung epithelial cell line. The HOS cell line is a human osteosarcoma cell line. TE671, MV-1-Lu and HOS cells can each be used as packaging cells for, for example, recombinant retroviruses such as murine leukemia viruses (MLV).

In a particular preferred embodiment, the engineered packaging cell line is derived from a HEK293T cell line. HEK293T cells are easy to handle, can be easily transfected using standard protocols and have a good transfection efficiency. As mentioned above, the wild-type HEK293T cell line is a commonly used packaging cell line for producing recombinant virus particles, in particular retrovirus particles, to date. Therefore, the use of an engineered cell line that is derived from a HEK293T cell line will provide benefits to the production of a broad range of recombinant virus particles.

In a preferred embodiment, the cell line is a HEK293T cell line in which the *B3GAT3* gene encoding the B3GAT3 protein is inactivated. The inventor found that a HEK293T cell line in which the *B3GAT3* gene, which encodes the B3GAT3 protein, is inactivated facilitates the production of viral supernatants with a high titer of recombinant virus particles even when non-cationic transfection reagents are used. Surprisingly, the inventor found that up to 100-fold higher virus vector titers can be obtained upon using a HEK293T packaging cell line in which the *B3GAT3* gene is inactivated compared to using the parental HEK293T packaging cell line.

The inventor also found that B3GAT3-deficient HEK293T packaging cell lines can be transfected by non-cationic or cationic polymer-based transfection reagents at similar efficiencies as parental HEK293T cells. Therefore, a B3GAT3-deficient HEK293T packaging cell line can be used for the production of any kind of virus vector that can employ the parental HEK293T cells.

In a preferred embodiment, the cell line is deposited under number DSM ACC3355 or DSM ACC3356. These cell lines, which have been generated by the inventor, are deposited at the following depositary institution:
Leibniz Institute DSMZ-German Collection of Microorganisms and Cell Cultures, Braunschweig, Germany.

For both cell lines, the date of the deposit is June 19, 2019. Both cell lines are engineered HEK293T cell lines in which the *B3GAT3* genes encoding the B3GAT3 protein are inactivated by CRISPR/Cas9 mediated genome editing. The engineered HEK293T single cell clones obtained were subject to selection by limiting dilution (HEK293T-25A; Accession number of the deposited cell line: DSM ACC3355) or single cell sorting of heparan sulfate-negative cells (HEK293T-306; Accession number of the deposited cell line: DSM ACC3356). For both clones, the absence of cell surface heparan sulfate expression was confirmed by flow cytometry as described above.

The guideRNA vector of the CRISPR/Cas9 system used by the inventor was designed to express a red fluorescent protein. The two engineered HEK293T cell clones that were selected as described above do not show any red fluorescence. The absence of the red fluorescence of the cell clones as such facilitates detection of a red fluorescent reporter protein, such as DsRed, during virus production in case a vector encoding a respective reporter protein is used. The detection of the reporter protein allows evaluating the transfection efficiency of the packaging cells by fluorescence microscopy before harvesting the virus particles.

In a preferred embodiment, the virus is selected from the group consisting of a retrovirus, a spumavirus, a lentivirus, an adenovirus, an adeno-associated virus, a herpesvirus, a human papillomavirus (HPV), a hepatitis virus, a Dengue virus (DENV), a Japanese encephalitis virus (JEV), a yellow fever virus (YFV), a togavirus, a filovirus, an orthobunyavirus and a rabies virus (RABV). The use according to the invention is particularly suitable for the production of these viruses since these viruses are known to bind to or to interact with cell surface GAGs, in particular cell surface heparan sulfate, on the surface of their target cells. According to the inventor's findings, by using a packaging cell line that is engineered to lack cell surface expression of GAGs, recombinant virus particles of these viruses can no longer be retained and/or adsorbed to the cell surface of the packaging cells in case non-cationic transfection reagents are used for virus production. Therefore, viral supernatants with a high titer of recombinant virus particles can be produced. In addition, transfection reagents other than cationic polymer-based transfection reagents can be employed.

In a preferred embodiment, the retrovirus is a spumavirus, preferably prototype foamy virus (PFV). Spuma- or foamy viruses (FV) are a special type of retroviruses that have been grouped into several genera of a separate retrovirus subfamily, the spumaretrovirinae, as a consequence of unique features in their replication strategy. Foamy virus is a particularly promising virus for gene transfer in therapeutic approaches in humans since it can infect almost every eukaryotic cell type and has the capacity to package large mRNA amounts both specific and unspecific. In addition, foamy virus is apparently apathogenic in vivo. The inventor found a particularly high increase in virus vector titers of recombinant foamy virus particles when using a packaging cell line that is engineered to lack cell surface expression of heparan sulfate compared to using the corresponding parental packaging cell line, even when non-cationic transfection reagents are used.

In another preferred embodiment, the retrovirus is a lentivirus, preferably human immunodeficiency virus (HIV), more preferred HIV type 1 (HIV-1).

In another preferred embodiment, the retrovirus is a gammaretrovirus, preferably murine leukemia virus (MLV).

In another preferred embodiment, the retrovirus is an alpharetrovirus, preferably Rous sarcoma virus (RSV).

In a preferred embodiment, the adeno-associated virus (AAV) is AAV2 or AAV6. The inventor found that the release of recombinant AAV particles from B3GAT3 deficient HEK293T packaging cells was increased compared to parental HEK293T packaging cells.

In a preferred embodiment, the herpesvirus is Herpes simplex virus (HSV), more preferred HSV-1, HSV-2 or cytomegalovirus (CMV).

In a preferred embodiment, the hepatitis virus is hepatitis B virus (HBV) or hepatitis C virus (HCV).

In a preferred embodiment, the DENV is DENV2.

In a preferred embodiment, the togavirus is chikungunya virus (CHIKV);

In a preferred embodiment, the filovirus is Ebola virus (EBOV) or Marburg virus (MARV).

In a preferred embodiment, the orthobunyavirus is Akabane virus (AKAV) or Schmallenberg virus (SBV).

In a preferred embodiment, the virus particle comprises a foamy virus envelope glycoprotein (FV Env) or a vesicular stomatitis virus glycoprotein (VSV-G), preferably the virus particle comprises a FV Env. In a further preferred embodiment, the virus is a retrovirus that comprises a FV Env.

The FV Env may be, for example, of the genus of *Simiispumaviruses* of Ape origin such as the prototype foamy virus (PFV, SFVpsc_huHSRV.13) isolate, or of Old World monkey origin such as the macaque simian foamy virus (macaque SFV, SFVmcy_FV21) isolate, or of New World monkey origin such as the spider monkey simian foamy virus (SFVspm, SFVaxx_Hooks40) isolate. As further examples, the FV Env may be derived from the genus of *Prosimiispumaviruses* of Prosimian origin such as the brown greater galago prosimian foamy virus (SFVgal, SFVocr_1557) isolate; or the FV Env may be derived from the genus of *Chispumaviruses* of Chiropteran origin such as the chiropteran foamy virus Rhinolophus affinis (CFVraf_1) isolate. As further examples, the FV Env may be derived from the genus of *Felispumaviruses* of Feline origin such as the cat feline foamy virus (FFV, FFVfca_F17/951) isolate; or the FV Env may be derived from the genus of *Bovispumaviruses* of Bovine origin such as the bovine foamy virus (BFV, BFVbta_Riems) isolate; or the FV Env may be derived from the genus of *Equispumaviruses* of Equine origin such as the equine foamy virus (EFV, EFVeca_1) isolate. The taxonomy and nomenclature used is based on Khan et al. (Khan et al. 2018).

In a preferred embodiment, the FV Env is the FV Env of prototype foamy virus (PFV) or the FV Env of macaque simian foamy virus (macaque SFV).

In a preferred embodiment, the FV Env is the FV Env of feline foamy virus (FFV), the FV Env of bovine foamy virus (BFV), the FV Env of equine foamy virus (EFV), or the FV Env of an gorilla foamy virus variant.

The glycoprotein can be the natural envelope glycoprotein of the virus. For example, the virus can be PFV and comprise the FV Env of PFV. Alternatively, the virus particle can comprise a viral envelope glycoprotein that is different from its natural envelope proteins (pseudotyped virus particle). For example, the virus can be HIV or MLV pseudotyped with a FV Env or pseudotyped with VSV-G. In another example, the virus can be PFV pseudotyped with FV Env of macaque SFV or pseudotyped with VSV-G. Pseudotyping of virus particles is commonly used to alter the tropism of the virus particles.

In a preferred embodiment, the cell line is engineered to lack cell surface expression of GAGs. In this case, the cell surface lacks all types of GAGs, i.e. (i) heparin and heparan sulfate GAGs, (ii) chondroitin sulfate and dermatan sulfate GAGs, (iii) keratan sulfates and (iv) hyaluronic acid.

In a second aspect, the present invention relates to a method for producing recombinant virus particles, the method comprising the steps of:
(a) transfecting a plurality of cells of a mammalian packaging cell line engineered to lack cell surface expression of heparan sulfate with
   (i) a transfer vector, and
   (ii) at least one viral packaging plasmid;
   thereby obtaining a plurality of producer cells;
(b) culturing the producer cells in a medium, thereby obtaining the virus particles in the medium; and
(c) separating the medium from the producer cells.

The method of the invention is an *in vitro* method that is based on the inventor's finding that the use of a mammalian packaging cell line engineered to lack cell surface expression of heparan sulfate facilitates the production of viral supernatants with a high titer of recombinant virus particles even when non-cationic transfection reagents are used. Surprisingly, the inventor found that up to 100-fold higher virus vector titers can be obtained when using a packaging cell line that is engineered to lack cell surface expression of heparan sulfate compared to using the corresponding parental packaging cell line.

In addition, since the method is suitable for obtaining high titer viral vector supernatants using non-cationic transfection reagents, viral vector supernatants that are devoid of remnants of cationic polymer-based transfection reagents can be produced. This is advantageous since remnants of cationic polymer-based transfection reagents in viral vector supernatants were found to act as inhibitory factor that reduces viral vector infectivity, in particular in certain types of target cells such as myeloid and lymphoid target cells.

The method thus facilitates the production of high titer recombinant virus particles with superior transduction efficiency in great variety of target cells, in particular in myeloid and lymphoid cells.

The method of the invention is technically easy to perform. The transfection and culture of the packaging cell line engineered to lack cell surface expression of heparan sulfate is generally the same as for the corresponding parental (wild-type) cell line. Apart from the engineered cell line used, the method of the invention follows a well established routine for producing recombinant virus particles.

First, the cells are transfected with all components required for viral packaging. The term "transfer vector" as used herein refers to the vector encoding the nucleic acid that is to be delivered by the virus to the target cells. The transfer vector can also be referred to as transfer vector plasmid, transfer plasmid or expression plasmid. The nucleic acid that is to be delivered may be, for example, a transgene (such as a therapeutic gene or a reporter gene) or an RNA molecule. In case another cargo molecule such as a heterologous viral protein, a non-viral protein, a nucleoprotein complex, a sugar or a drug is to be delivered by the virus to the target cells, the term "transfer vector" as used herein refers to the vector encoding the respective cargo molecule or encoding the production machinery for the respective cargo molecule.

In addition to the transfer vector, at least one viral packaging plasmid harbouring the elements required for packaging and encapsulation of the cargo molecule needs to be provided to the cells. Typically, for example for producing recombinant retrovirus particles, two or three viral packaging plasmids are used. For retrovirus particles, one packaging plasmid may comprise the Gag and Pol genes, and, if applicable, the Rev gene, while a different packaging plasmid may comprise the Env gene. Alternatively, as for example in a state-of-the-art FV vector system, three packaging plasmids for the viral structural components Gag, Pol and Env are used, each preferably in an expression-optimized version.

Transfection conditions may be adjusted, for example depending on the transfection reagent employed, the type of cell line used and/or the type of virus.

The term "producer cells" as used herein refers to the transfected packaging cells.

During the culture of the producer cells, the cells produce virus particles and release the virus particles into the medium in which the cells are cultivated. In this way, the virus particles are obtained in the medium. Most cells are cultivated at a temperature of 37°C, a CO₂ concentration of 5% and a relative humidity of 95% in a suitable medium (cell culture medium). Cell culture conditions may be adjusted, for example depending on the type of cell line used.

After a sufficient period of time for virus production (for example about 48, 72 or 96 hours), the medium (supernatant) is separated from the producer cells. This separation step serves to harvest the virus particles produced. The separation step can be performed by commonly used techniques such as collecting the medium and preferably centrifuging the collected medium to pellet any producer cells that may have been taken up during medium collection. In this way, cell-free (i.e. released) virus particles are harvested.

The separation step can alternatively be performed by freeze-thawing of the producer cells together with the medium and subsequent removal of cell debris by low-speed centrifugation and/or sterile filtration. In this way, cell-bound virus particles are also harvested.

Optionally, the virus particles may then be recovered from the medium. The term "recovery" as used herein refers to the concentration of the medium in which the virus particles are present in order to obtain a higher virus titer. The recovery or concentration step can be performed by commonly used techniques such as ultracentrifugation or ultrafiltration.

In a preferred embodiment, the virus is selected from the group consisting of a retrovirus, a spumavirus, a lentivirus, an adenovirus, an adeno-associated virus, a herpesvirus, a human papillomavirus (HPV), a hepatitis virus, a Dengue virus (DENV), a Japanese encephalitis virus (JEV), a yellow fever virus (YFV), a togavirus, a filovirus, an orthobunyavirus and a rabies virus (RABV).

In a preferred embodiment, the retrovirus is a lentivirus, preferably human immunodeficiency virus (HIV), more preferred HIV type 1 (HIV-1), or the retrovirus is a spumavirus, preferably prototype foamy virus (PFV), or the retrovirus is a gammaretrovirus, preferably murine leukemia virus (MLV), or the retrovirus is an alpharetrovirus, preferably Rous sarcoma virus (RSV).

In a preferred embodiment, the hepatitis virus is hepatitis B virus (HBV) or hepatitis C virus (HCV).

In a preferred embodiment, the cell surface expression of heparan sulfate is lacking due to the packaging cell line being engineered not to have a functional heparan sulfate synthesis pathway.

In a preferred embodiment, the packaging cell line is engineered to lack an active B3GAT3 protein. The active B3GAT3 protein is lacking preferably due to the cell line being genetically engineered to lack expression of the B3GAT3 protein.

In a preferred embodiment, the cell line is derived from a cell line selected from the group consisting of HEK293T cell line, HEK293 cell line, HeLa cell line, D-17 cell line, HT1080 cell line, TE671 cell line, MV-1-Lu cell line and HOS cell line. In a preferred embodiment, the cell line is derived from a HEK293T cell line.

In a particular preferred embodiment, the cell line is a HEK293T cell line in which the B3GAT3 gene encoding the B3GAT3 protein is inactivated.

In a preferred embodiment, the cell line is deposited under number DSM ACC3355 or DSM ACC3356.

In a preferred embodiment, step (a) is performed by using a non-cationic transfection reagent, preferably calcium phosphate. The use of calcium phosphate for the transfection of cells involves mixing the DNA that is to be transferred with calcium chloride in a buffered saline/phosphate solution in order to generate a calcium-phosphate-DNA co-precipitate (calcium phosphate coprecipitation). The co-precipitate is then added to the cells. Calcium phosphate facilitates the binding of the condensed DNA in the co-precipitate to the surface of a cell, and the DNA enters the cell by endocytosis.

The inventor found that when using a packaging cell line that is engineered to lack cell surface expression of heparan sulfate for the production of FV Env-comprising recombinant virus particles, virus vector titers obtained by using non-cationic transfection reagents are up to 10-fold higher compared to virus titers obtained by using cationic polymer-based transfection reagents. In addition, the use of non-cationic transfection reagents results in the absence of remnants of cationic polymer-based transfection reagents in the viral vector supernatants. As described above, such remnants were found to act as inhibitory factor that reduces viral vector infectivity, in particular in certain types of target cells such as myeloid and lymphoid target cells. A reduction of viral vector infectivity is not observed when non-cationic transfection reagents are used.

The packaging cell line that is engineered to lack cell surface expression of heparan sulfate is suitable for using non-cationic transfection reagents as well as cationic polymer-based transfection reagents. Therefore, in another embodiment, step (a) is performed by using a cationic polymer-based transfection reagent such as polyethylenimine. Polyethylenimine is a cationic polymer that can bind and condensate DNA. DNA-polyethylenimine-complexes are readily endocytosed by a variety of cell lines. The inventor found that the use of cationic polymer-based transfection reagents for transfection of the engineered packaging cell line results in similar FV Env-comprising virus vector titers as obtained with the corresponding parental packaging cell line.

In a preferred embodiment, the transfer vector is a viral vector, i.e. a vector that comprises viral sequences. For retrovirus particles, the viral sequences of the transfer vector are typically long terminal repeat (LTR) regions that are flanking the nucleic acid sequence that is to be delivered by the virus to the target cells. Viral vectors are commonly used as transfer vectors.

In another embodiment, the transfer vector is a non-viral vector, i.e. a vector that is devoid or substantially devoid of any viral sequences. A suitable non-viral transfer vector is, for example, the so-called minimal retrovirus vector system described in WO 2012/152632 A1. The minimal retrovirus vector system does not have any foamyviral sequences in the transfer vector and thus in the packaged RNA that is transferred into the target cells. It can thereby facilitate a transient genetic modification of the target cells. The only viral sequence present in the transfer vector of the minimal retrovirus vector system is a cytomegalovirus (CMV) immediate early 1 enhancer promoter that is used for transcription of the mRNA which is then packaged in the recombinant virus particles. The inventor found that recombinant virus particles obtained by using such a non-viral transfer vector can also be produced at high titers when a cell line engineered to lack cell surface expression of heparan sulfate is used. More specifically, the inventor produced the virus particles in a B3GAT3 deficient HEK293T cell line using calcium phosphate coprecipitation for the transfection of the cells. The virus particles obtained showed a very good transduction efficiency on human myeloid cells. The inventor also found that when producing the virus particles in the B3GAT3 deficient HEK293T cell line using calcium phosphate (or other non-cationic transfection reagents) for transfection, inhibitory effects of cationic polymer-based transfection reagents such as polyethylenimine on certain target cell types such as monocytes are avoided.

The production of the recombinant virus particles harbouring a non-viral transfer vector in the B3GAT3 deficient HEK293T cell line using calcium phosphate coprecipitation for the transfection of the cells was found to yield higher virus titers compared to production in the wild-type HEK293T cell line.

When comparing the use of cationic polymer-based or non-cationic transfection reagents for the production of recombinant virus particles harbouring a non-viral transfer vector in a packaging cell line that is engineered to lack cell surface expression of heparan sulfate, the inventor found that virus titers obtained by using a non-cationic transfection reagent such as calcium phosphate are comparable to virus titers obtained by using cationic polymer-based transfection reagents such as polyethylenimine.

In a third aspect, the present invention relates to a recombinant virus particle obtainable by the method of the invention. As a result of producing the virus particle in cells lacking cell surface expression of heparan sulfate, the surface composition of the virus particle that is released from the cells can be different from a surface composition of a virus particle released from a cell that is positive for cell surface expression of heparan sulfate.

In a further aspect, the present invention relates to a mammalian packaging cell line deposited under number DSM ACC3355 or DSM ACC3356. Both cell lines are engineered HEK293T cell lines in which the *B3GAT3* gene encoding the B3GAT3 protein is inactivated by CRISPR/Cas9 mediated genome editing. The cell lines and their advantages have been described in more detail above.

In a further aspect, the present disclosure relates to the use of an engineered mammalian packaging cell line for producing a stable packaging cell line for producing recombinant virus particles, wherein the cell line is engineered to lack cell surface expression of heparan sulfate. The term "stable packaging cell line" as used herein refers to a cell line that stably expresses viral packaging components such as the viral structural components Gag, Pol and Env. In order to produce recombinant virus particles, the stable packaging cell line may be transiently transfected with a transfer vector. Alternatively, the stable packaging cell line may stably express the transfer vector.

FV Env proteins are very fusogenic and readily induce cell-cell fusion if stably expressed in cells or if high amounts of FV Env-comprising virus particles are added to target cells. The inventor found that FV Env-mediated infectivity und thus FV Env-mediated cytotoxic effects are considerably reduced in case a packaging cell line engineered to lack cell surface expression of heparan sulfate, such as a B3GAT3-deficient cell line, is used. The cell line that is engineered to lack cell surface expression of heparan sulfate is therefore particularly suitable for generation of a stable packaging cell line for producing FV Env-comprising virus particles.

In a further aspect, the present disclosure relates to a method for producing recombinant virus particles, the method comprising the steps of:
(a) transfecting a plurality of cells of a stable mammalian packaging cell line engineered to lack cell surface expression of heparan sulfate with
   (i) a transfer vector,
   thereby obtaining a plurality of producer cells;
(b) culturing the producer cells in a medium, thereby obtaining the virus particles in the medium; and
(c) separating the medium from the producer cells.

In a further aspect, the present disclosure relates to the use of an engineered mammalian cell line for producing recombinant non-viral complexes having binding sites for heparan sulfate, wherein the cell line is engineered to lack cell surface expression of heparan sulfate. The binding sites for heparan sulfate may be natural or artificially introduced binding sites. By using a cell line that is engineered to lack cell surface expression of heparan sulfate, the recombinant non-viral complexes will no longer be retained and/or adsorbed to the cell surface of the cells. Therefore, the recombinant non-viral complexes can be produced at high titers.

The non-viral complexes are preferably complexes that can be used for transferring one or more nucleic acids or other cargo molecules such as proteins, nucleoprotein complexes, sugars or drugs to or into a target cell. The non-viral complexes can be particulate structures (particles).

The non-viral complexes can be, for example, vesicles or vesicle-like particles. The vesicles can be natural vesicles (such as exosomes) or artificial vesicles. The vesicles can be loaded with the cargo that is to be transferred by the vesicles. The same applies to vesicle-like particles. The non-viral complexes can be, for example, artificial viruses or virus-like particles. Artificial viruses or virus-like particles have a capsid-like structure.

As mentioned above, the non-viral complexes are preferably complexes that can be used for transferring a molecule of interest, for example a therapeutic molecule, to or into a target cell. A suitable non-viral complex for this purpose is, for example, the so-called enveloped protein nanocage having the vesicular stomatitis virus glycoprotein on its surface, which is described in Votteler et al. (Votteler, J. et al. 2016). This non-viral complex can be produced in HEK293T cells for example and is released into the culture medium of the cells. This non-viral complex can be regarded as an artificial virus.

In a preferred embodiment, the non-viral complexes are exosomes. Exosomes are extracellular vesicles that are produced in the endosomal compartment of most eukaryotic cells and that are then released by the cells. Exosomes are an interesting potential vehicle for the delivery of therapeutic molecules to target cells.

In a further aspect, the present disclosure relates to a method for producing recombinant non-viral complexes having binding sites for heparan sulfate, the method comprising the steps of:
(a) transfecting a plurality of cells of a mammalian cell line engineered to lack cell surface expression of heparan sulfate with one or more nucleic acids required to produce the non-viral complex,
   thereby obtaining a plurality of producer cells;
(b) culturing the producer cells in a medium, thereby obtaining the non-viral complexes in the medium; and
(c) separating the medium from the producer cells.

In a further aspect, the present disclosure relates to a recombinant non-viral complex having binding sites for heparan sulfate obtainable by the method disclosed. As a result of producing the non-viral complex in cells lacking cell surface expression of heparan sulfate, the surface composition of the non-viral complex that is released from the cells can be different from a surface composition of a non-viral complex released from a cell that is positive for cell surface expression of heparan sulfate.

In a further aspect, the present disclosure relates to the use of an engineered mammalian packaging cell line for producing recombinant virus particles, wherein the cell line is engineered to lack cell surface expression of chondroitin sulfate and/or dermatan sulfate. Depending on the envelope protein of recombinant virus particles, chondroitin sulfate and/or dermatan sulfate may play a role in the release of the recombinant virus particles into the cell culture supernatants of packaging cells as was found for heparan sulfate and FV Env-comprising virus particles. Therefore, a cell line that is engineered to lack cell surface expression of chondroitin sulfate and/or dermatan sulfate will be useful for producing recombinant virus particles of viruses that primarily interact with cell surface chondroitin sulfate and/or dermatan sulfate, respectively.

As mentioned above, like heparan sulfate, chondroitin sulfate and dermatan sulfate are present at the cell surface in the form of proteoglycans. Accordingly, the cell line may be engineered to lack one or more active enzymes of the chondroitin sulfate and/or dermatan sulfate proteoglycan synthesis pathway. The engineered cell line may have a functional heparan sulfate proteoglycan synthesis pathway.

In a further aspect, the present disclosure relates to a method for producing recombinant virus particles, the method comprising the steps of:
(a) transfecting a plurality of cells of a mammalian packaging cell line engineered to lack cell surface expression of chondroitin sulfate and/or dermatan sulfate with
   (i) a transfer vector, and
   (ii) at least one viral packaging plasmid;
   thereby obtaining a plurality of producer cells;
(b) culturing the producer cells in a medium, thereby obtaining the virus particles in the medium; and
(c) separating the medium from the producer cells.

In a further aspect, the present disclosure relates to a recombinant virus particle obtainable by the method disclosed.

Further aspects of the invention will be apparent to the person skilled in the art by the enclosed description of the examples, in particular the scientific results.

### Examples

### Materials and methods

### CRISPR/Cas9 system

The CRISPR/Cas9 system is a genetic engineering tool capable to introduce a double-strand break at a precisely defined target region. It was used in combination with a foamy virus (FV) vector system to target the *B3GAT3* gene in the genome of HEK293T cells. The targeting efficiency of CRISPR/Cas9 was measured by a genotypic analysis of the target region (T7 endonuclease assay) and a detection of the resulting phenotype, namely the presence or absence of heparan sulfate (HS) on cell surfaces by an indirect fluorescence antibody staining followed by flow cytometry.

### Cas9 expression constructs

The Cas9 expression construct pcoCas9-T2A-GFP harbours a codon-optimized version of the *SpCas9* ORF with N-terminal Flag and SV40 nuclear localization signal (NLS) tag, C-terminal nucleoplasmin NLS tag and downstream C-terminal in frame fusion of a T2A peptide coding sequence and *EGFP* ORF and out of frame woodchuck hepatitis virus posttranscriptional regulatory element (WPRE), all inserted into the CMV promoter/enhancer and intron A driven eukaryotic expression vector pczi (Heinkelein, M. et al. 2002). Upon translation, the T2A peptide will cause a ribosomal skipping at the spliced mRNA between Cas9 and *EGFP* ORFs resulting in two separate proteins for Flag-NLS-Cas9-NLS and GFP to be expressed. Cas9 protein fusion to a NLS facilitates the import of the nuclease into the nucleus of the target cell.

### sgRNA expression constructs

Vectors for sgRNA (single-guiding RNA) expression are based on replication-deficient prototype foamy virus (PFV) transfer vector "puc2MD9" (replication-deficient PFV transfer vector comprising all necessary cis-active sequences (CAS) of PFV *gag-* and pol-ORF) harboring a human U6 promoter driven expression cassette for sgRNA and a spleen focus forming virus U3 promoter driven reporter gene (*DsRed Express* 2) expression cassette. Several versions of the vector containing different B3GAT3 specific sgRNA were created.

### Inactivation of the B3GAT3 gene

Parental, wild-type HEK293T cells were transduced with Cas9 encoding PFV RNA transfer vector (TraFo) supernatants in combination with various integration-competent PFV vector supernatants encoding U6 promoter-driven B3GAT3-specific sgRNA expression cassettes or respective controls. Two and nine days post transduction (p.t.), cell surface heparan sulfate (HS) expression of the individual cell populations was examined by immunostaining and flow cytometry. The three cell populations with the strongest reduction in cell surface HS expression were used at day 14 p.t. to set up for subcloning of cell clones by limiting dilutions in 96-well plates. Furthermore, one population was chosen on day 21 p.t. for immunostaining with anti-HS antibodies under sterile conditions and HS-negative cells were single-cell sorted into 96-well plates. Individual clones were expanded and subsequently screened by flow cytometric analysis of HS cell surface expression at the 24-well stage 16 to 27 days after initiating single-cell cloning in 96-well plates. HS cell surface negative clones were further expanded, HS cell surface expression of individual clones was reanalyzed at least once, and stock aliquots frozen at the 10 cm dish stage and stored in liquid nitrogen. HS negative single cell clones were only obtained from population 10, one from the limiting dilution and more than 10 from the single-cell sorting approach. Two clones (293T-25A, clone 10-4E2; 293T-306, clone 10-1F10) did not express DsRed Ex2, indicating that they lack a stably integrated B3GAT3 sgRNA expression cassette. These clones were chosen for further characterization with respect to their properties as viral packaging cell lines.

### Single cell cloning

Treating a cell population with a CRISPR/Cas9 system results in a genetically heterogeneous population of cells. To obtain monoclonal cell populations, single cells have to be isolated, expanded and screened. In this work, this was achieved by either a serial dilution (limiting dilution) or flow cytometric cell sorting. All monoclonal cell colonies resulting from one of the two methods were expanded and screened by immunofluorescence staining and flow cytometry analysis.

### Limiting dilution

The limiting dilution aimed at seeding a final concentration of 0.5 cells/well into a 96-well plate. To achieve this, a cell population was diluted down in six subsequent dilution steps. First, cells were detached from culture surfaces, suspended in DMEM (10% FCS) and counted using a Neubauer counting chamber. A first dilution of the estimated cell concentration to 1x 10⁵ cells/ml was performed, and the exact concentration of the resulting solution was determined again using the Neubauer chamber. If the measured concentration did not match to 1x 10⁵ cells/ml, samples were diluted as necessary to gain the desired concentration. After this confirmatory step, samples were diluted further in four sequential steps to 1 x 10⁴, 1 x 10³, 1 x 10² and finally, 1x 10¹ cells/ml. In a last step, 12.5 ml of the obtained 1x 10¹ cells/ml solution were mixed with 37.5 ml DMEM (10% FCS), which results in a concentration of 2.5 cells/ml. This final dilution was seeded into a 96-well plate at 200 µl/well. This way, each well contained a theoretic amount of 0.5 cells/well. The outer rows and columns of each plate were filled to the rim with PBS to prevent a rapid evaporation of the small volumes.

Cells were then incubated at 37°C to allow for cell expansion.

All wells were microscopically inspected periodically after seeding. Wells were marked according to the actual number (0, 1 or more than 1) of cells contained. Later, monoclonal colonies were transferred to a larger culture dish (24-well plates) to continue expansion. The time of transfer was individual for each colony and adjusted to their perceived size and speed of growth.

### Cell sorting

Cell sorting was used to sort HEK293T cells infected with CRISPR/Cas9 components according to their fluorescence characteristics. Infected cells were stained as described in section "Immunofluorescence labelling". The FACS buffer used for cell sorting did not contain sodium azide. Cell sorting was done using a FACS Aria II (Becton-Dickinson) device. Stained cells were measured by FACS and sorted into 96-well plates at a concentration of 1 cell/well. Treatment continued as for cells singled out by limiting dilution (see section above).

### T7 endonuclease assay

A T7 endonuclease assay is a method to detect on-target CRISPR/Cas9 events in a cell population. It relies on the capacities of T7 endonuclease I, isolated from the bacteriophage T7, to detect and cleave heteroduplex or nicked DNA. If a target region is recognized by the CRISPR/Cas9 System, a double-strand break is introduced by the Cas9 protein. In absence of a homologous piece of DNA, the repair NHEJ (non-homologous end joining) mechanism sprouts into action which will often result in an indel mutation at the position of the cut. A T7 endonuclease assay takes place in 4 steps: (i) amplification of target regions, (ii) annealing of DNA, (iii) T7 digest and (iv) gel electrophoresis. In the first step, the target region is amplified by PCR; double strands of resulting fragments are then denatured and reannealed in the second step, which causes the formation of heteroduplex DNA (if a strand amplified from an on-target CRISPR/Cas9 event pairs with a wild-type (wt) strand). During T7 endonuclease I digest, those incidents of imperfectly matched DNA are detected and cleaved by the enzyme. The resulting multi-length fragments of DNA can subsequently be analysed by gel electrophoresis.

**Reaction conditions of T7 endonuclease annealing step:**

| Compound | Volume |
|---|---|
| DNA Template (PCR) | 4 µl |
| 10x NEB Buffer 2 | 2 µl |
| ddH2O | 12 µl |
| ∑ | 19 µl |

**Cycling protocol for annealing reaction:**

| Step | Temperature | Ramp Rate | Time |
|---|---|---|---|
| Initial denaturation | 95°C | | 5 min |
| Annealing | 95-85°C | -2°C/s | |
| | 85-25°C | -0.1°C/s | |
| Cool down | 4°C | | hold |

An additional purification step after the initial PCR reaction by gel electrophoresis and gel extraction may be added. In the present work, another control mechanism was applied. After PCR, 5 µl of samples were loaded onto an agarose gel and run at 120 V for 1 h. Simultaneously, the annealing reaction was initiated. If the desired fragment was clearly visible on the agarose gel, 1 µl of T7 endonuclease I (New England BioLabs, Frankfurt a.M., Germany) was added to annealed samples in order to continue to the step of T7 digest. The digest took place at 37°C for 15 min. A control sample without the enzyme, but ddH2O instead was carried along for all amplifications. After digest, the whole sample (20 µl) was loaded on an agarose gel for analysis. Since the expected fragments were very small (< 500 bp), agarose gels were prepared with 2% agarose.

### Cultivation of cells

Both adherent cells (HEK293T, 293T-25A, 293T-306, HT1080, U2OSgfp, Pac2, MouseL, Sog9) and cells growing in suspension (Jurkat, THP-1) were used.

Adherent cells were cultivated in 175 or 75 cm² cell culture flasks in an incubator (37°C, 5% CO2, 95% humidity). Most adherent cell lines were grown in Dulbecco's Modified Eagle Medium (DMEM) complete medium (Thermo Fisher Scientific or Invitrogen), with the exception of Pac2 cells (Leibowitz's medium; Invitrogen). Cells were passaged at least twice a week (three times in the case of HEK293T cells) to prevent density-dependent inhibition of proliferation. For passaging, cells were detached from culture vessel surface by applying Trypsin-EDTA solution to the monolayer and incubation at 37°C for 5-10 min. The reaction was stopped by adding fresh complete medium. Cells were then homogenized by resuspending and reseeded in culture vessels at the required density (mostly 1:20).

Suspension cells were cultivated under similar conditions to adherent cells, but with RPMI complete medium (Thermo Fisher Scientific or Invitrogen). To passage cells, which was done twice a week, cells were homogenized by resuspending and reseeded in culture vessels at the required density (mostly 1:5).

All work with cells and cell lines was conducted under sterile conditions.

### Flow cytometry analysis

Fluorescence activated cell sorting (FACS) is a type of flow cytometry that specifically examines the fluorescence profiles of measured cells. In this work, the device "FACS Calibur" from Becton-Dickinson, Heidelberg, Germany, was used (except for cell sorting, for which a FACS Aria II from Becton-Dickinson was used). Fluorochromes used in this work are DsRed (emission maximum at 583 nm), PE (emission maximum at 573 nm), Alexa488 (emission maximum at 525 nm) and EGFP (emission maximum at 510 nm). They were measured using the FACS Calibur filters FL-1 (EGFP and Alexa488) with a wavelength of 530 nm and a bandwidth of 30 nm and FL-2 (DsRed and PE) with a wavelength of 585 nm and a bandwidth of 42 nm.

Results from FACS analyses were analysed using the softwares CellQuest Pro and FlowJo (both Becton-Dickinson, Heidelberg, Germany).

### Immunofluoresence labelling

In this work, an indirect fluorescence staining approach was used. Since the molecule of interest, HS, is present in the form of heparan sulfate proteoglycan at the cell surface, cells didn't have to be permeabilized and fixed prior to antibody staining, and even live cell staining (in the context of cell sorting) with subsequent re-seeding of cells was possible. Cells (1x 10⁵) were firstly detached from culture surfaces (in case of adherent cultures) using only PBS-EDTA solution without trypsin, since the latter is known to destroy proteoglycan molecules on cell surfaces. Both cells derived from adherent or suspension cultures were then seeded in FACS tubes containing 2 ml FACS staining buffer (PBS with 5% (v/v) FCS and 0.1% (w/v) sodium azide) and centrifuged (1,200 rpm, 5 min). After discarding of the supernatant, cell pellets were resuspended in 100 µl primary antibody (solution containing 0.1 µg antibody diluted in 100 µl FACS buffer) and incubated on ice for 30 min. After the first incubation, a washing step was executed by suspending cells in 4 ml FACS Buffer and centrifuging them again (1,200 rpm, 5 min). The supernatant was discarded and cell pellets resuspended in 100 µl secondary antibody (solution containing 0.1 - 0.175 µg antibody diluted in 100 µl FACS buffer). The secondary antibody was incubated for 30 min on ice and in the dark, so the bleaching effect on fluorochromes would be minimized. As a last step, cells were washed again and re-suspended in 300 µl FACS buffer for analysis via flow cytometry.

In all experiments, three different controls were carried along: (1) cells stained with an isotype antibody and secondary antibody, (2) cells stained with only secondary antibody, and (3) unstained cells. During incubation steps, the according antibody solution was replaced by plain FACS buffer.

### Transfection of packaging cells using calcium phosphate

The day before transfection, 5x 10⁶ HEK293T cells were seeded into 10 cm culture dishes containing 10 ml DMEM (10% FCS) and incubated at 37°C. Cells should be 80-90% confluent before continuing (24 h). Before transfection, the complete culture medium was exchanged by 8 ml DMEM (10% FCS) pre-equilibrated at 37°C, 5% CO₂ for at least 1 hour. The transfection mix was set up by rapid addition of 1 ml 2x HBS solution [50 mM Hepes (pH 7.05), 10 mM KCl, 12 mM Dextrose-H₂O, 280 mM NaCl, 1.5 mM Na₂HPO₄] to 1 ml DNA solution (30 µg of total DNA, 124 µl 2M CaCl₂ and ddH₂O in total volume of 1 ml) in 2 ml Eppendorf tubes and subsequent rapid mixing by vortexing for 10 sec. After 30 sec at room temperature, the transfection mix was added carefully but swiftly dropwise to the HEK293T cells in 10 cm dishes. After 6-8 h of incubation at 37°C, 5% CO₂, the transfection mixture was aspirated and replaced by 8 ml fresh DMEM (10% FCS). In cases where sodium butyrate (NaBu) stimulation was employed, NaBu was added at 24 h post transfection to 10 mM final concentration and incubation continued at 37°C, 5% CO₂. At 30 to 32 h post transfection, cells were deprived of old culture medium, washed with 5 ml PBS and covered with 6 ml new culture medium DMEM (10% FCS) per 10 cm dish. After overnight incubation at 37°C, 5% CO₂, at 44 h to 48 h post transfection, virus particles were ready to be harvested.

### Transfection of packaging cells using polvethylenimine (PEI)

The day before transfection, 5x 10⁶ HEK293T cells were seeded into 10 cm culture dishes containing 10 ml DMEM (10% FCS) and incubated at 37°C. Cells should be 80-90% confluent before continuing (24 h). Before transfection, a media change was executed on seeded cells and the complete culture medium exchanged by 4 ml DMEM (15% FCS). The transfection mix was set up by mixing 16 µg of total DNA with 48 µg PEI in a total volume of 2 ml DMEM plain and incubating for about 20 min. The transfection mix was added to culture dishes carefully. Transfected cells were incubated overnight at 37°C. After approximately 30 h, cells were deprived of old culture medium, washed with 5 ml PBS and covered with 6 ml new culture medium DMEM (10% FCS). After a second night of incubation, virus particles were ready to be harvested (44 h to 48 h after transfection).

### Harvest of viral particles

To harvest cell-free virus supernatant, the medium in a culture dish was aspirated with a syringe and subsequently filtered through 0.45 µm syringe sterile filter or transferred into Falcon tubes and cleared by low-speed centrifugation (600x g, 5 min, room temperature). The virus-containing solution was either used for a direct infection of target cells or snap frozen and stored at -80°C for later use.

Cell-bound virus supernatants were obtained by a single freeze-thaw cycle. After removal of the growth medium to harvest virus particles released into the supernatant (cell-free virus supernatant), as described above, 6 ml fresh culture medium DMEM (10% FCS) was added to the cell layer in the 10 cm dish. Subsequently, the dishes with medium were placed either on a metal plate sitting on dry ice for about 10 min or into a -80°C freezer for about 20 min, until fully frozen. Thereafter, the frozen dishes were placed into a tissue culture incubator (37°C, 5% CO₂) until the liquid was fully thawed. Next the solution was gently but thoroughly mixed by pipetting up and down before transfer into Falcon tubes and centrifugation (5 min, 1,000x g, 4°C) to pellet cellular debris. Finally, the supernatant, except for a small volume above the debris pellet, was transferred to a fresh tube, aliquoted and directly used or stored as described above.

**Amount and kind of DNA used for PEI transfection:**

| Compound | 4-component FV vector system | 3-component FV vector system | 1-component FV proviral expression system | 3-component HIV or MLV vector system |
|---|---|---|---|---|
| Transfer vector | 11.4 µg | 12 µg | | 7 µg |
| Provirus expression plasmid | | | 16 µg | |
| Gag packaging plasmid | 2.6 µg | 3.2 µg | | |
| Pol packaging plasmid | 1.2 µg | | | |
| Gag/Pol packaging plasmid | | | | 7 µg |
| Env packaging plasmid | 0.8 µg | 0.8 µg | | 1-2 µg |
| ∑ | 16 µg | 16 µg | 16 µg | 15-16 µg |

### PFV packaging plasmids

pcoPG4: Eukaryotic expression plasmid for codon-optimized PFV gag, CMV enhancer/promoter, based on pcDNA 3.1 zeo⁺, includes CMV intron A for expression of spliced mRNAs.
pcoPP: Eukaryotic expression plasmid for codon-optimized PFV pol, CMV enhancer/promoter, based on pcziPol, includes CMV intron A for expression of spliced mRNAs.
pcoPE: Eukaryotic expression plasmid for codon-optimized PFV env, CMV enhancer/ promoter, based on pcDNA 3.1 zeo⁺, includes CMV intron A for expression of spliced mRNAs.
pcoSE: Eukaryotic expression plasmid for codon-optimized SFVmac env, CMV enhancer/promoter, based on pcDNA 3.1 zeo⁺, includes CMV intron A for expression of spliced mRNAs.

### HIV or MLV packaging plasmids

pCD/NL-BH: Eukaryotic expression plasmid for HIV-1 Gag/Pol, Rev, CMV enhancer/promoter, based on pcDNA.
pHIT60: Eukaryotic expression plasmid for MLV Gag/Pol, CMV enhancer/promoter, based on pBR322.
pcoPE01: Eukaryotic expression plasmid for codon-optimized PFV env variant for efficient pseudotyping, CMV enhancer/promoter, based on pcDNA 3.1 zeo⁺, includes CMV intron A for expression of spliced mRNAs.
pcoSE03: Eukaryotic expression plasmid for codon-optimized SFVmac env variant for efficient pseudotyping, CMV enhancer/promoter, based on pcDNA 3.1 zeo⁺, includes CMV intron A for expression of spliced mRNAs.

### Infection of target cells

Fresh or frozen virus particles were used for the infection (transduction) of target cells. Adherent cells were seeded at a density of 2.5x 10⁴ per well (in a 12-well plate, 1 ml growth (culture) medium per well) and incubated overnight at 37°C. The following day, the culture medium was aspirated and replaced with virus-containing solution in an end volume of 1 ml. Virus supernatants were used undiluted or diluted as indicated. If different virus solutions were combined, the ratio was 1:1 if not stated otherwise. After approximately 5 h of incubation, the virus supernatant was replaced by the same volume of fresh growth medium. Usually, cells were used for further experiments (generally FACS analysis) at 72 h after infection (or 2-3 days post-infection).

For transductions of suspension cells, per well 1x 10⁵ cells in 50 µl volume were added to 500 µl undiluted or diluted virus supernatant in 24-well plates. When undiluted supernatants were employed, virus supernatant incubation was terminated 4-6 hours later by transfer of individual samples into 1.5 ml Eppendorf tubes, pelleting the cells by centrifugation (600x g, 5 min, room temperature), discarding the supernatant, gentle resuspension of the cell pellet in 500 µl fresh growth medium, and transfer into a fresh 24-well plate, followed by incubation at 37°C, 5% CO₂ until further analysis as indicated. When only virus dilutions (10⁻¹ or lower) were employed, virus supernatants were left on the cells until further analysis at later time points, generally at 48-72 h post infection.

### Western blotting

For Western blot analysis of viral particle protein composition, about 10 ml cell free virus supernatant was carefully layered onto 2 ml 20% sucrose in ultracentrifuge tubes. Virus particles were pelleted by centrifugation in SW-32Ti rotors at 25,000 rpm (-82,600 gₐᵥₑ), at 4°C for 90 min. After discarding the supernatant, the virus pellet was resuspended in 1x protein sample buffer [40 mM Tris, pH 7.6; 8% (v/v) glycerol; 3% (w/v) SDS; 0.1 M DTT; 0.01% (w/v) Coomassie Blue G 250], boiled for 10 min at 95°C, and stored at -20°C until further use.

Protein lysates of packaging cells were obtained by washing the cell layer once with PBS after removal of the growth medium containing the released virus particles. After aspiration of the PBS, 600 µl cold 1x lysis buffer [10 mM Tris, pH 8.0; 140 mM NaCl; 0.025 NaN₃; 1% (v/v) Triton X-100] per 10 cm dish was added to the cell layer and incubated on a rocking platform for 20 min at 4°C. Subsequently, cells were scraped of the dish using a rubber policeman and the whole suspension transferred to QIAshredder in a 2 ml Eppendorf tube. After centrifugation at 13,000 rpm for 2 min, 4°C in a table top centrifuge, 600 µl 2x protein sample buffer was added to the QIAshredder, followed by another 2 min centrifugation step. Both eluates were combined, gently mixed and boiled at 95°C for 10 min, and stored at -20°C until further use.

Aliquots of virus and/or cell protein lysates were loaded onto commercial precast or selfmade 7.5% Tricine PAGE. After separation in an electric field, the proteins were transferred to nitrocellulose membranes by semidry blotting. After blocking of nitrocellulose membranes in PBS-T (PBS, 0.5% Tween 20), 5% skim milk for 2 h, membranes were washed twice briefly with PBS-T. Subsequently, membranes were incubated with primary antibody solution in PBS-T, 5% skim milk, for 1 h to overnight at room temperature or 4°C, followed by two brief and three 5 min washes in PBS-T. Next, second appropriate horse radish peroxidase (HRP) coupled 2^{nd} step reagents in PBS-T, 5% skim milk were added and incubated for 1 h at room temperature. After two brief and three 10 min washes in PBS-T and careful removal of all excess liquid the membrane was incubated with chemoluminescent HRP substrate for 1 min. Finally, after removal of excess substrate, chemoluminescent signals of membranes wrapped in Saran wrap were digitally recorded using a Fuji LAS-3000 bioimager and LAS software V2.2.

### Adeno-Associated Virus vector production

Recombinant AAV (rAAV) vector particles were generated by transient transfection of the respective packaging cell lines using Lipofectamine LTX with PLUS Reagent technology. Briefly, 1.6x 10⁶ cells were plated in T-75 flasks two days prior to transfection. Equal amounts (3 µg) of pAAV-YFG transfer vector as well as pRC and pHelper packaging plasmids (9 µg total DNA) were added to 3.75 ml OptiMEM medium in a tube. Nineteen µl of PLUS reagent were added, gently mixed and the mixture incubated for 5 to 10 min at room temperature. Subsequently, 47 µl of Lipofectamine LTX were added to the tube, gently mixed, and the mixture incubated for 30 min at room temperature. In the meantime, the T-75 flask with the adherent packaging cells was washed twice with PBS, 7.5 ml OptiMEM was added and the cells preincubated for at least 15 min at 37°C, 5% CO₂. Subsequently the DNA/Lipid transfection mixture was gently added to the T-75 flask containing the packaging cells pre-equilibrated in OptiMEM and incubated at 37°C, 5% CO₂ for 4 to 6 h. Next, the transfection mixture medium was aspirated and replaced by 10 ml fresh DMEM (10% FCS) and cultivation at 37°C, 5% CO₂ continued. Two days later rAAV was harvested from cell-free supernatants and freeze-thaw lysates of packaging cells resuspended in fresh DMEM (10%) using three freeze-thaw cycles, vortexing and removal of cellular debris by centrifugation at 10,000 g for 30 min at 4°C. Virus supernatants were aliquoted and stored at - 80°C until further use.

### Results

Before the scientific results are described in detail, it is referred to Fig. 1 which visualizes some key findings of the inventor in relation to the present invention. First it was found that free heparan sulfate binding sites on the cell surface of wild-type HEK293T (293T wt) packaging cells retain FV Env-comprising virus particles upon transient transfection of the cells with non-cationic transfection reagents. This results in low cell-free viral vector titers and therefore poor target cell transduction (Panel 1). In contrast, cationic polymer-based transfection reagents neutralize cell surface heparan sulfate binding sites on 293T wt packaging cells. Thereby high cell-free viral vector titers are achieved (Panel 2). However, remnants of the polymer-based transfection reagents present in viral vector supernatants interfere with the transduction of target cells by blocking target cell heparan sulfate binding sites, thereby diminishing virus particle attachment to target cells and reducing gene transfer efficiency (Panel 2). The same is true if cationic polymer-based transfection reagents are employed for FV Env-comprising virus particle production using HEK293T packaging cells that are engineered to lack an active B3GAT3 protein (293T ΔB3GAT3) and that are therefore deficient in cell surface GAG expression (Panel 3). In contrast, the use of non-cationic transfection reagents in combination with 293T ΔB3GAT3 packaging cells allows the production of high titer viral vector supernatants (Panel 4). Potential remnants of non-cationic transfection reagents in the vector supernatants do not interfere with heparan sulfate-dependent attachment of FV Env-comprising virus particles to target cells and thereby allow efficient gene transfer into a great variety of target cells (Panel 4).

The experimental results will now be described in detail.

### Differential Susceptibility of Human Fibroblast and Monocyte Cell Lines towards Single-Round Retrovirus Vector Particles comprising FV Env

For many types of target cells, the transduction efficiency of recombinant viral vector particles comprising foamy virus envelope glycoprotein (FV Env) is superior to viral vector particles comprising other viral glycoproteins such as vesicular stomatitis virus glycoprotein (VSV-G). However, this advantage is not observed for certain target cell types such as myeloid cells and lymphoid cells (see for example Fig. 2).

Fig. 2 shows the result of vector titration experiments. HT1080 (human fibroblast cell line) and THP-1 (human monocyte cell line) cells were incubated with decreasing serial dilutions of GFP encoding retroviral vector particles of human immunodeficiency virus (HIV), murine leukemia virus (MLV) and prototype foamy virus (PFV) origins pseudotyped with different viral glycoproteins as indicated in Fig. 2 (VSV: vesicular stomatitis virus glycoprotein; SE: macaque simian foamy virus; PE: prototype foamy virus). Fig. 2 shows the percentage of GFP expressing cells in the individual samples determined seventy-two hours post infection (p.i.) by flow cytometry. Shown is a representative experiment (n=3). The results showed that viral vector infectivity of FV Env-comprising viral supernatants is reduced when higher virus doses are applied to THP-1 target cells. Based on this finding, the inventor assumed that the FV Env-comprising viral supernatants produced by current state of the art methods harbour an inhibitory factor that reduces viral vector infectivity when higher virus doses are applied to target cells.

### Infectivity Inhibition by Mock HEK293T Supernatants of Different Composition

THP-1 target cells were incubated with decreasing serial dilutions of recombinant GFP encoding HIV vector particles pseudotyped with VSV-G (Fig. 3A, HIV - VSV) or SFVmac (Fig. 3B, HIV - SE) glycoproteins that were mixed at a 1:1 ratio with mock supernatants of different composition, as indicated in Fig. 3 (PEI: polyethylenimine). Mock supernatant concentration was constant at 1/12^{th} of total. Fig. 3 shows the percentage of GFP expressing cells in the individual samples determined seventy-two hours post infection (p.i.) by flow cytometry. The results showed decreasing HIV - SE viral vector particle infectivity at constant mock supernatant concentration in the individual samples if mock supernatant contained PEI (293T medium + PEI; 293T medium + PEI-DNA). In contrast, HIV - VSV particle infectivity was not affected by any type of mock supernatant tested.

A calculation of the potential residual PEI concentration in the individual samples due to residual PEI in the viral supernatants was based on the assumptions that (i) there is a homogenous distribution of PEI in the transfection mix and in the cell-free viral supernatant, and (ii) there are 500 µl of carry-over solution during each of the two washing steps. The resulting estimates of PEI concentration in the transfection mix and in the viral supernatant are:

| | |
|---|---|
| Transfection mix: | 8 µg/ml |
| Viral supernatant: | 56 ng/ml |

Based on the calculation of the potential residual PEI concentration in the individual samples, the inventor concluded that remnants of the cationic polymer-based transfection reagents such as PEI in the viral vector supernatants are the inhibitory factor that reduces viral vector infectivity when higher virus doses are applied to certain types of target cells (Fig. 2).

### Incomplete Neutralization of PEI-Contamination-Mediated Inhibition of FV Env Infectivity by Virus Purification

Only the use of protocols employing HEK293T (293T) packaging cells in combination with cationic polymer-based transfection reagents allows the production of high titer supernatants of viral vector particles comprising FV Env to date (see e.g. Fig. 13). Therefore, the inventor attempted to remove remnants of the cationic polymer-based transfection reagents, which were found to reduce the transduction efficiency of certain target cell types (Fig. 2 and 3), by additional post production purification steps such as low speed- or ultracentrifugation, ultrafiltration and size exclusion chromatography.

To do so, THP-1 target cells were incubated with decreasing serial dilutions of GFP encoding HIV vector particles pseudotyped with VSV-G (HIV - VSV) or SFVmac (HIV - SE) glycoproteins that beforehand were further purified, as indicated in Fig. 4. The abbreviations are as follows: P: plain 293T supernatants; UC: virus particles pelleted by ultracentrifugation; UC+UF: virus particles pelleted by ultracentrifugation and subsequently concentrated by ultrafiltration; SEC: virus particles purified by size exclusion chromatography. Fig. 4 shows the percentage of GFP expressing cells in the individual samples determined seventy-two hours post infection (p.i.) by flow cytometry. It was found that the attempts to remove remnants of the cationic polymer-based transfection reagents by additional post production purification steps were largely unsuccessful (Fig. 4).

### Comparison of PFV and lentiviral (HIV-1) vector titers obtained by transient transfection of various 293T based packaging cell lines

The inventor generated modified clonal 293T packaging cells with a defective GAG synthesis pathway by CRISPR/Cas9 mediated inactivation of the *B3GAT3* gene. The inventor isolated single cell clones of the B3GAT3 deficient (ΔB3GAT3) 293T cells and confirmed that the cells did not show cell surface expression of heparan sulfate as determined by flow cytometry analysis (see Fig. 11).

The inventor performed a detailed experimental comparison of the use of parental (wild type) 293T (wt) and two representative proteoglycan-deficient ΔB3GAT3 293T (25A, 306) packaging cell lines for the production of FV Env (either PFV [PE] or SFVmac [SE])-comprising, replication-deficient retroviral vectors based on prototype foamy virus (PFV) or human immunodeficiency virus type 1 (HIV-1). Vector supernatants were generated by calcium phosphate (CaPO) [Ca₃(PO₄)₂] co-precipitation or polyethylenimine (PEI) transfection in parallel in parental 293T (wt) cells or one of the two B3GAT3-deficient 293T cell clones (25A, 306).

In detail, parental 293T (wt) or proteoglycan-deficient 293T variant (25A, 306) packaging cell lines were transiently transfected with expression plasmid encoding for either a replication-deficient, expression optimized 4-component PFV vector system or a replication-deficient 3-component HIV-1 vector system using PEI or CaPO transfection reagents. Glycoprotein packaging plasmids employed encoded the PFV Env protein (PE, PE01), macaque SFV Env protein (SE, SE03), or the VSV glycoprotein (VSV). Subsequently, infectivity of plain, unconcentrated, cell-free supernatants was determined on HT1080 target cells using a flow cytometric EGFP transfer assay 72 hours post infection (p.i.). Fig. 5A, left panel, shows the mean values and standard deviations (StdDev; n=3) of absolute viral titers on HT1080 target cells (uninf: uninfected control). Fig. 5A, right panel, shows the infectivity of supernatants relative to PFV vector supernatants comprising PFV Env and derived by transient transfection of parental 293T (wt) packaging cells using calcium phosphate transfection technique. Fig. 5B, left panel, shows the fold difference of viral titers of individual samples relative to PFV vector supernatants comprising PFV Env and derived by transient transfection of parental 293T (wt) packaging cells using calcium phosphate transfection technique (uninf: uninfected control). Fig. 5B, right panel, shows the fold difference of viral titers of the individual samples relative to the type of virus vector derived by transient transfection of parental 293T (wt) packaging cells.

The values underlying the graphs of Fig. 5A and 5B are as follows (Transf. Meth., transfection method):

| **Transf. Meth.** | **293T** | **Virus Type** | **Env** | **fold difference relative to 293T wt** | | **relative infectivity [%]** | | **Titer [EGFP ffu/ml]** | |
|---|---|---|---|---|---|---|---|---|---|
| | | | | **Mean** | **StdDev** | **Mean** | **StdDev** | **Mean** | **StdDev** |
| CaPO | wt | PFV | PE | 1.00 | 0.00 | 100.00% | 0.0% | 3.5E+04 | 1.8E+04 |
| | | | SE | 1.00 | 0.00 | 492.21% | 115.7% | 1.7E+05 | 9.0E+04 |
| | | HIV | VSV | 1.00 | 0.00 | 19684.88% | 8804.7% | 6.1 E+06 | 1.4E+06 |
| | | | PE01 | 1.00 | 0.00 | 75357.31% | 44865.4% | 2.1E+07 | 5.3E+06 |
| | | | SE03 | 1.00 | 0.00 | 140794.45% | 55522.3% | 4.5E+07 | 1.5E+07 |
| | 25A | PFV | PE | 67.56 | 33.14 | 6755.79% | 3314.1% | 2.1 E+06 | 1.0E+06 |
| | | | SE | 20.85 | 6.82 | 9956.50% | 2832.7% | 3.2E+06 | 8.3E+05 |
| | | HIV | VSV | 0.52 | 0.09 | 10607.36% | 6507.1% | 3.1 E+06 | 9.0E+05 |
| | | | PE01 | 3.23 | 1.65 | 214731.09% | 132154.6% | 6.3E+07 | 1.8E+07 |
| | | | SE03 | 2.04 | 0.56 | 266132.05% | 19735.2% | 9.2E+07 | 4.2E+07 |
| | 306 | PFV | PE | 47.33 | 14.91 | 4733.38% | 1490.9% | 1.7E+06 | 1.1E+06 |
| | | | SE | 19.96 | 4.36 | 9501.77% | 595.0% | 3.3E+06 | 1.5E+06 |
| | | HIV | VSV | 0.47 | 0.02 | 9278.43% | 4151.5% | 2.9E+06 | 7.6E+05 |
| | | | PE01 | 2.05 | 0.86 | 138089.85% | 76234.6% | 4.1 E+07 | 7.0E+06 |
| | | | SE03 | 1.55 | 0.35 | 205317.72% | 26906.0% | 7.0E+07 | 3.2E+07 |
| PEI | wt | PFV | PE | 1.00 | 0.00 | 4168.13% | 1024.3% | 1.5E+06 | 6.8E+05 |
| | | | SE | 1.00 | 0.00 | 3764.41% | 2928.9% | 1.0E+06 | 2.4E+05 |
| | | HIV | VSV | 1.00 | 0.00 | 7045.23% | 2932.4% | 2.5E+06 | 1.9E+06 |
| | | | PE01 | 1.00 | 0.00 | 121072.70% | 90155.1% | 3.6E+07 | 2.2E+07 |
| | | | SE03 | 1.00 | 0.00 | 70766.63% | 46928.3% | 3.0E+07 | 3.2E+07 |
| | 25A | PFV | PE | 1.40 | 0.49 | 5687.26% | 1712.6% | 1.8E+06 | 4.4E+05 |
| | | | SE | 1.50 | 0.31 | 5113.43% | 2862.9% | 1.5E+06 | 1.1E+05 |
| | | HIV | VSV | 1.77 | 0.55 | 11462.66% | 3013.6% | 3.9E+06 | 1.8E+06 |
| | | | PE01 | 1.32 | 1.15 | 92284.92% | 36852.3% | 3.7E+07 | 2.9E+07 |
| | | | SE03 | 1.68 | 0.58 | 102871.06% | 31927.9% | 3.9E+07 | 3.0E+07 |
| | 306 | PFV | PE | 0.96 | 0.54 | 4309.17% | 3358.0% | 1.6E+06 | 1.3E+06 |
| | | | SE | 1.87 | 0.63 | 8268.75% | 8885.6% | 2.0E+06 | 1.1E+06 |
| | | HIV | VSV | 1.71 | 0.80 | 11196.26% | 6554.5% | 3.3E+06 | 4.3E+05 |
| | | | PE01 | 1.16 | 0.70 | 103772.04% | 36682.2% | 3.3E+07 | 1.3E+07 |
| | | | SE03 | 2.79 | 2.50 | 143943.83% | 75207.5% | 4.5E+07 | 1.8E+07 |
| | | | uninf | 0.00 | 0.00 | 0.25% | 0.1% | 7.2E+01 | 0.0E+00 |

First, the results confirmed several observations made by the inventor with respect to the use of the parental 293T (wt) packaging cell line: (i) The production of high titer viral vector supernatants comprising FV Env proteins by transient transfection of 293T packaging cells is strongly dependent on the type of transfection reagent used. (ii) Transfection protocols employing standard non-cationic transfection reagents such as calcium phosphate, which is frequently used for production of vesicular stomatitis virus glycoprotein (VSV-G) pseudotypes of lentiviral vectors, only yields very poor titers of FV Env-comprising retroviral vectors. Calcium phosphate coprecipitation results in 10- to 100-fold lower viral vector supernatant titers compared to cationic polymer-based transfection reagents. (iii) In contrast, transfection reagents based on cationic polymers like PEI or Polyfect (Qiagen) yield up to 100-fold higher FV Env-comprising viral vector titers, whereas no significant difference in vector titers is observed for lentiviral VSV-G pseudotypes.

More importantly, the results revealed that the ΔB3GAT3 293T packaging cell lines (25A, 306) can be transfected by non-cationic or cationic polymer-based transfection reagents at similar efficiencies as parental 293T cells (wt). This is demonstrated by similar virus titers obtained for VSV-G containing vector particles. Therefore, the ΔB3GAT3 293T packaging cell lines can be used for the production of any kind of virus vector that can employ the parental 293T wild type cells.

Surprisingly, it was found that FV Env-comprising, replication-deficient, infectious PFV vector titers obtained by the calcium phosphate coprecipitation transfection methods using ΔB3GAT3 293T-25A (25A) or ΔB3GAT3 293T-306 (306) cells are 20-fold (SE) to 60-fold (PE) higher than that using parental 293T (wt) cells (Fig. 5B). In contrast, similar vector titers were obtained for all three 293T packaging cell lines by PEI transfection (Fig. 5B). Titers of PFV vector supernatants of ΔB3GAT3 293T-25A and ΔB3GAT3 293T-306 cells produced by calcium phosphate transfection were slightly higher than those generated by PEI transfection (Fig. 5B).

It was further found that FV Env-comprising, replication-deficient HIV-1 vector titers obtained by the calcium phosphate coprecipitation transfection methods using ΔB3GAT3 293T-25A or ΔB3GAT3 293T-306 cells are slightly higher (SE 1.5-fold to 2.0-fold higher; PE: 2.1-fold to 3.2-fold higher) than that using parental 293T (wt) cells and similar to that obtained by the PEI transfection method using all types of 293T cells (Fig. 5B).

### Comparison of replication-competent PFV titers and physical particle release obtained by transient transfection of various 293T based packaging cell lines

The inventor further performed an experimental comparison of the use of parental 293T (wt) and proteoglycan-deficient ΔB3GAT3 293T (25A) packaging cell lines for the production of replication-competent wild type PFV.

Parental 293T (wt) or proteoglycan-deficient 293T variant (25A) packaging cell lines were transiently transfected with PFV proviral expression constructs using PEI or CaPO transfection methods with (+) or without (-) sodium butyrate (NaBu) induction. In Fig. 6A physical particle release was determined by Western blot analysis of samples of PFV particles concentrated and purified by ultracentrifugation followed by SDS-PAGE and immunoblotting using PFV Gag and PFV Env LP subunit specific polyclonal antisera. In Fig. 6B the infectivity of supernatants was determined on HT1080 PLNE target cells harboring a PFV transactivator Tas-dependent EGFP reporter gene expression cassette using a flow cytometric assay at 24 h post infection. Fig. 6B, left panel, shows mean values and StdDev (n=4) of absolute viral titers of replication-competent viral supernatants on HT1080 PLNE target cells. Fig. 6B, right panel, shows relative infectivity in comparison to virus supernatants produced by CaPO mediated transient transfection of proteoglycan-deficient 293T-25A (25A) packaging cells.

The values underlying the graph of Fig. 6B are as follows (Transf. Meth., transfection method):

| **Transf. Meth.** | **293T** | **NaBu** | **relative infectivity [%]** | | **Titer [EGFP ffu/ml]** | |
|---|---|---|---|---|---|---|
| | | | **Mean** | **StdDev** | **Mean** | **StdDev** |
| CaPO | 25A | + | 100.00% | 0.0% | 6.1 E+05 | 2.2E+05 |
| | | - | 77.71% | 55.5% | 4.8E+05 | 4.5E+05 |
| | wt | + | 0.99% | 0.3% | 5.8E+03 | 1.8E+03 |
| | | - | 0.50% | 0.2% | 3.0E+03 | 1.6E+03 |
| PEI | 25A | + | 16.29% | 9.2% | 8.7E+04 | 3.7E+04 |
| | | - | 11.74% | 6.7% | 6.9E+04 | 5.2E+04 |
| | wt | + | 12.71% | 7.2% | 6.8E+04 | 3.1E+04 |
| | | - | 8.32% | 4.6% | 4.7E+04 | 2.8E+04 |
| | mock | | 0.01% | 0.0% | 7.2E+01 | 0.0E+00 |

Surprisingly, Western Blot analysis revealed a more than 27-fold higher physical particle release by calcium phosphate transfection using 293T-25A (25A) rather than parental 293T (wt) cells (Fig. 6A).

Also surprisingly, titration analysis on HT1080 PLNE cells revealed that virus titers of replication-competent PFV viral supernatants obtained by calcium phosphate coprecipitation transfection methods using ΔB3GAT3 293T-25A (25A) cells are 100-fold higher than that using parental 293T (wt) cells and 5-fold to 10-fold higher than that obtained by the PEI transfection method using either 293T-25A (25A) or parental 293T (wt) cells (Fig. 6B).

### Retroviral Vector Infectivity Inhibition/Enhancement on Different Target Cells by Exogenous PEI Addition

Different target cells were incubated with decreasing serial dilutions of GFP encoding HIV vector particles pseudotyped with VSV-G (HIV - VSV) or SFVmac (HIV - SE) glycoproteins that had fixed concentrations of exogenously added PEI, as indicated in Fig. 7. The HIV vector particles were obtained using parental (wild type) 293T packaging cells using PEI transfection technique. Fig. 7 shows the mean values and StdDev (n=3) of the percentage of GFP expressing cells in the individual samples determined seventy-two hours post infection (p.i.) by flow cytometry. The insets in the graphs relating to VSV-G infected Mouse L and Sog9 cells (Fig. 7D and 7E) show the FACS staining profiles of heparan sulfate cell surface expression of the respective cell lines (Isotype Ctrl: isotype control). It is known that Sog9 cells are deficient in heparan sulfate due to defects in cellular heparan sulfate proteoglycan (HSPG) synthesis.

The results showed a dose-dependent inhibition of FV Env mediated viral infectivity under specific conditions, namely for the combination of viral vector supernatants produced by PEI transfection of 293T wt cells and certain target cells types such as the human monocyte cell line THP-1 or the human T-cell line Jurkat (Fig. 7A and 7B; for THP-1 cells see also Fig. 2). The unique infection phenotype (also referred to as "PEI phenotype" herein), which is observed by titration of viral supernatants on these cell lines, is characterized
(i) in case of SFVmac Env by a very low infection rate at the highest virus doses examined, followed first by an increase in the infection rate with serial dilution of the virus supernatant before it is then decreasing upon further serial dilution (Fig. 2, Fig. 3B, Fig. 4, Fig. 7A, Fig. 7B, Fig. 10, Fig. 12B, and Fig. 14), or
(ii) in case of PFV Env by a very low infection rate at the highest virus doses examined, which stays at a constant low level in the next serial dilution of the virus before it is then decreasing upon further serial dilution (Fig. 2, Fig. 10, and Fig. 12B).

The inventor found that this is due to the presence of residual PEI contaminations in the viral vector supernatant (Fig. 3). Furthermore, FV Env but not VSV-G mediated particle infectivity could be inhibited in a dose-dependent manner by the addition of PEI to vector supernatants on many target cell types, except those with defects in cellular heparan sulfate proteoglycan synthesis (Fig. 7, Fig. 8).

### Dependence of Retroviral Vector Infectivity Inhibition/Enhancement by Exogenous PEI Addition on Target Cell Heparan Sulfate Cell Surface Expression

Cell surface heparan sulfate (HS) positive wild-type 293T (293T wt) or cell surface HS deficient 293T-25A target cells were incubated with decreasing serial dilutions of GFP encoding HIV vector particles pseudotyped with VSV-G, PFV (PE) or SFVmac (SE) glycoproteins that had fixed concentrations of exogenously added PEI, as indicated in Fig. 8. The HIV vector particles were generated by transient transfection of proteoglycan-deficient ΔB3GAT3 293T-25A packaging cells using calcium phosphate transfection technique. Fig. 8 shows the mean values and StdDev (n=3) of the percentage of GFP expressing cells in the individual samples determined seventy-two hours post infection (p.i.) by flow cytometry. The results showed that FV Env-mediated viral vector infectivity is inhibited in a dose-dependent manner by the addition of PEI to vector supernatants in case of transduction of cell surface HS positive target cells (293T wt) whereas transduction of cell surface HS negative target cells (293T-25A) is not affected by exogeneous PEI addition.

### Reduced Permissiveness of B3GAT3-deficient 293T cells towards FV Env-pseudotyped retroviral vector particles

Wild-type 293T cells (wt) or B3GAT3-deficient 293T cell variants (25A, 306) were incubated with decreasing serial dilutions of GFP encoding PFV or HIV vector particles harboring VSV-G (HIV - VSV), PFV (PFV - PE; HIV - PE) or SFVmac (PFV - SE; HIV - SE) glycoproteins, as indicated in Fig. 9. The PFV and HIV vector particle supernatants were generated by transient transfection of proteoglycan-deficient HEK293T ΔB3GAT3 (293T-25A) packaging cells using calcium phosphate transfection technique. Fig. 9 shows the mean values and StdDev (n=3) of the percentage of GFP expressing cells in the individual samples determined seventy-two hours post infection (p.i.) by flow cytometry. The results showed a 50-fold to 100-fold reduced infection efficiency of B3GAT3-deficient 293T cell lines 293T-25A (25A) and 293T-306 (306) in comparison to the parental 293T (wt) cells. This demonstrates an intrinsic reduced permissiveness (susceptibility) of B3GAT3-deficient (proteoglycan-deficient) 293T cells (25A, 306) towards FV Env-pseudotyped retroviral vector particles compared to wild type 293T cells (wt) as a consequence of the lack of HS cell surface expression on B3GAT3-deficient 293T cell lines. This is also relevant during the production of recombinant virus particles with respect to potential re-absorption of virus particles by their packaging cells.

### Titration of PFV and HIV-1 retroviral vector supernatants harboring various viral glycoproteins and produced by calcium phosphate or polyethylenimine transfection using 293T-25A cells on THP-1 target cells

Cell-free PFV (PFV) and HIV-1 (HIV) single-round vector supernatants harboring various viral glycoproteins (VSV: vesicular stomatitis virus glycoprotein; PE: PFV envelope glycoprotein; SE: SFVmac glycoprotein) were produced by transient transfection of B3GAT3-deficient 293T-25A packaging cells using the respective EGFP encoding transfer vector and packaging construct combination and calcium phosphate (CaPO) or polyethylenimine (PEI) transfection methods as indicated in Fig. 10. Subsequently, THP-1 target cells were incubated with decreasing serial dilutions of the respective vector supernatants as indicated in Fig. 10. Fig. 10 shows the percentage of GFP expressing cells in the individual samples determined six days post infection (p.i.) by flow cytometry. Shown is a representative experiment (n=3). The results showed that the typical "PEI phenotype" described above (Fig. 7) was observed for PEI-derived FV Env-pseudotyped retroviral vector supernatants. In contrast, for calcium phosphate derived FV Env-pseudotyped retroviral vector supernatants, a dose-dependent increase in the fraction of infected THP-1 cells was observed with the use of increasing amounts of viral vector supernatant.

Regarding the typical "PEI phenotype" of PEI-derived HIV-PE vector supernatants, a very low infection rate, namely an infection rate of less than 10% (about 8%), was observed at the three highest virus doses examined. Accordingly, more than 90% of the target cells were not infected. As mentioned above, this is due to the inhibitory effect of residual PEI contaminations in the viral vector supernatants. Calcium phosphate derived HIV-PE vector supernatants, in contrast, showed an infection rate of about 43%. Thus, calcium phosphate derived HIV-PE vector supernatants showed a more than 5-fold increase in virus vector titers compared to PEI-derived HIV-PE vector supernatants.

Regarding the typical "PEI phenotype" of PEI-derived HIV-SE vector supernatants, an infection rate of PEI-derived HIV-SE vector supernatants of 8.1% compared to 74.56% for calcium phosphate derived HIV-SE vector supernatants was observed at the highest virus dose examined. Thus, calcium phosphate derived HIV-SE vector supernatants showed a more than 9-fold increase in virus vector titers compared to PEI-derived HIV-SE vector supernatants.

### Cell surface heparan sulfate expression of 293T packaging cell variants

Flow cytometry analysis of cell surface heparan sulfate (HS) expression of wild-type HT1080 cells (HT1080 wt), parental wild-type 293T cells (293T-wt) and 293T single cell clones, obtained after CRISPR/Cas9 mediated B3GAT3 gene inactivation, followed by limiting dilution (293T-25A) or single cell sorting of HS-negative cells (293T-306). Single cell suspensions were stained with monoclonal mouse anti-HS antibody (F58-10E4; Amsbio, Abingdon, Great Britain) or mouse IgM K isotype control (mlgM Isotype Ctrl; BD Biosciences, Heidelberg, Germany) and goat anti-mouse IgG+IgM (H+L) Alexa 488 conjugated (a-mouse IgM Alexa488; Jackson Immunoresearch) second step reagent as indicated in the legend of Fig. 11. Fig. 11 shows overlay histograms of mouse anti-HS (solid lines, grey area) or mouse IgM K isotype control stainings or further control samples as indicated in the legend (dashed lines, white area) of the individual cell types. The results confirmed that parental 293T cells (293T-wt) and HT1080 cells (HT1080 wt) express heparan sulfate on their cell surface. The results further confirmed that the B3GAT3-deficient 293T packaging cell variants (293T-25A, 293T-306) lack cell surface heparan sulfate expression.

### Comparison of physical particle release obtained by transient transfection of various 293T based packaging cell lines with components of different FV vector systems

Parental 293T (293T wt) or proteoglycan-deficient 293T variant (293T-25A) packaging cell lines were transiently transfected with PFV proviral expression constructs for production of replication-competent PFV (RCP), or expression constructs for production of *EGFP* encoding replication-deficient PFV single-round vector particles (SRV), or expression constructs for production of *EGFP* encoding replication-deficient PFV RNA Transfer vector particles (TraFo) harboring PFV Env (PE), SFVmac Env (SE) or VSV-G (VSV) as indicated, using polyethylenimine (PEI) or calcium phosphate (CaPO) transfection methods. Fig. 12A shows the physical particle release determined by Western blot analysis of samples of PFV particles concentrated and purified by ultracentrifugation followed by SDS-PAGE and immunoblotting using PFV Gag and PFV Env LP subunit specific polyclonal antisera. The results showed that for all three types of PFV particles a much higher physical particle release was obtained by calcium phosphate transfection using 293T-25A (25A) rather than parental 293T (wt) cells, if they comprised FV Env glycoproteins. Fig. 12B and 12C show the infectivity of supernatants determined on HT1080 target cells harboring a PFV transactivator Tas-dependent EGFP reporter protein expression cassette (HT1080-PLNE) or THP-1 target cells (THP-1) by incubation with decreasing serial dilutions of the respective vector supernatants as indicated. Forty-eight hours post infection (p.i.) the percentage of GFP expressing cells (%-GFP) in the individual samples or the mean fluorescent intensity of the viable cell population in the GFP channel (MFI-GFP) was determined by flow cytometry. Fig. 12B shows %-GFP positive cells of HT1080-PLNE or THP-1 cell samples incubated with serial dilutions of the same PFV-SRV supernatants harboring PE or SE Env, produced by CaPO or PEI transfection methods using proteoglycan-deficient 293T-25A packaging cells. Fig. 12C shows MFI-GFP of HT1080-PLNE or THP-1 cell samples incubated with serial dilutions of the same PFV TraFo supernatants harboring PE, SE Env, or VSV-G, produced by CaPO or PEI transfection methods using proteoglycan-deficient 293T-25A packaging cells. Shown are data from a single experiment. The results showed that in THP-1 cells, the typical "PEI phenotype" described above (Fig. 7) was observed for PFV-SRV supernatants harboring PE or SE Env as well as for PFV TraFo supernatants harboring PE or SE Env.

### Dependence of cell-free PFV vector titers on the transfection method utilized

Wild-type 293T packaging cells were transfected with two variants of a 2-component PFV vector system consisting of a PFV Gag & Pol expressing transfer vector harboring a spleen focus forming virus U3 region (SFFV U3) -driven LacZ reporter protein expression cassette (DWP02, MH120) and either a PFV Env packaging plasmid (+) or pCDNA3.1 (-). The cells were transfected using Polyfect or calcium phosphate (CaPO) transfection methods as indicated. Fourty-eight hours post transfection cell-free virus supernatants were harvested by sterile filtration of the medium and cell-bound virus supernatants by addition of fresh medium to the transfected packaging cells, a single freeze-thaw cycle and removing cell debris by centrifugation and sterile filtration. Fig. 13A and 13B show the examination of physical particle release as determined by Western blot analysis of lysates from packaging cells (cell) transfected with the 2-component PFV vector system expression constructs (Fig. 13A) using Polyfect and CaPO transfection methods as well as PFV particles (virus) concentrated and purified by ultracentrifugation from respective cell-free virus supernatants (Fig. 13B) followed by SDS-PAGE and immunoblotting using PFV Gag and PFV Env LP subunit specific polyclonal antisera. The results showed higher amounts of viral proteins (Gag and Env) present in cell-free virus lysates in case the packaging cells were transfected using Polyfect transfection method compared to CaPO transfection method (Fig. 13B). This is despite the fact that similar or even slightly higher cellular expression levels of PFV Gag and Env proteins in CaPO vs Polyfect transfected packaging cells were detectable (Fig. 13A). This indicates that Polyfect transfection leads to higher cell-free PFV vector titers. Fig. 13C shows the infectivity determined by a LacZ gene transfer assay. Serial dilutions of cell-free and cell-bound virus supernatants were incubated with HT1080 target cells. Seventy-two hours later LacZ expressing cells were visualized by histochemical β-galactosidase staining and counting of "blue" foci in individual wells (ffu, focus-forming units). Shown are mean values ± StdDev (n=3). The results showed that cell-bound PFV vector titers obtained by Polyfect or CaPO transfection of the packaging cells were similar, while cell-free PFV vector titers were considerably higher in case the packaging cells were transfected using Polyfect transfection method.

### Remnants of different cationic polymer-based transfection reagents in FV Env-comprising retroviral vector supernatants inhibit infection of THP-1 cells

GFP encoding HIV vector particles pseudotyped with VSV-G (HIV - VSV) or SFVmac (HIV - SE) glycoproteins were generated by transiently transfecting the respective expression plasmids of replication-deficient 3-component HIV-1 vector system into wild-type parental 293T packaging cells using cationic polymer-based transfection reagents, namely polyethylenimine (PEI) or Polyfect as indicated. HT1080 or THP-1 target cells were incubated with decreasing serial dilutions of the GFP encoding retroviral vector supernatants as indicated in Fig. 14. Fig. 14 shows the percentage of GFP expressing cells in the individual samples determined seventy-two hours post infection (p.i.) by flow cytometry. Shown are data from a representative experiment (n=2). The results showed that the use of Polyfect leads to a lower viral vector infectivity compared to PEI. The results further showed that in THP-1 cells, the typical "PEI phenotype" for HIV - SE described above (Fig. 7) was also observed in case Polyfect was used as transfection reagent. The results thus showed that remnants of PEI and Polyfect transfection reagents in FV Env-comprising retroviral vector supernatants inhibit infection of THP-1 cells.

### Enhanced cell-free rAAV vector release in B3GAT3-deficient packaging cells

Recombinant adeno-associated virus (rAAV) vector particles were generated by transient transfection of different packaging cell lines as indicated (293: wild-type HEK293 cell line; 293T: wild-type HEK293T cell line; 293T-25A: B3GAT3-deficient 293T cell line; uninf.: uninfected control) using the respective GFP encoding transfer vector and packaging plasmids. Cell-free rAAV vector particles (cell-free) released from the producer cells were harvested from the cell culture supernatant by low speed centrifugation. Cell-bound rAAV vector particles (cell-bound) retained within the packaging cells and released by freeze-thawing of producer cells in medium were harvested by high speed separation of the lysate and separation of the cellular debris pellet. Subsequently, infectivity of supernatants was determined on HT1080 target cells using a flow cytometric EGFP transfer assay. Fig. 15 shows the percentage of GFP positive cells determined four days post infection (p.i.) by flow cytometry. Shown are data from a single experiment. The results showed an enhanced cell-free rAAV vector release in B3GAT3-deficient packaging cells.

The values underlying the graph of Fig. 15 are as follows:

| **Supernatant Type** | **Packaging Cell** | **Relative Infectivity [%]** | | **Titer [EGFP ffu/ml]** | |
|---|---|---|---|---|---|
| | | **Mean** | **StdDev** | **Mean** | **StdDev** |
| cell-bound | 293 | 100.0% | 27.09% | 1.4E+06 | 3.7E+05 |
| | **293T** | 98.9% | 30.62% | 1.3E+06 | 4.1E+05 |
| | 293T-25A | 114.3% | 39.13% | 1.5E+06 | 5.3E+05 |
| cell-free | 293 | 66.0% | 11.47% | 8.9E+05 | 1.6E+05 |
| | 293T | 101.5% | 27.66% | 1.4E+06 | 3.7E+05 |
| | 293T-25A | 155.9% | 62.26% | 2.1E+06 | 8.4E+05 |
| | uninf. | 0.0% | 0.00% | 8.7E+01 | 0.0E+00 |

### References

Heinkelein, M. et al. Improved primate foamy virus vectors and packaging constructs. J. Virol. 2002;76(8):3774-83.
Jinek, M. et al. A programmable dual-RNA-guided DNA endonuclease in adaptive bacterial immunity. Science 2012;337:816-821.
Khan, A.S. et al. Spumaretroviruses: Updated taxonomy and nomenclature. Virology 2018;516:158-164.
Votteler, J. et al. Designed proteins induce the formation of nanocage-containing extracellular vesicles. Nature 2016;540:292-295.
WO 2012/152632 A1

## Claims

1. Use of an engineered mammalian packaging cell line for producing recombinant virus particles, wherein the cell line is engineered to lack cell surface expression of heparan sulfate.

2. The use according to claim 1, wherein the cell surface expression of heparan sulfate is lacking due to the cell line being engineered not to have a functional heparan sulfate proteoglycan synthesis pathway.

3. The use according to claim 1 or claim 2, wherein the cell line is engineered to further lack cell surface expression of chondroitin sulfate and dermatan sulfate.

4. The use according to claim 3, wherein the cell surface expression of heparan sulfate, chondroitin sulfate and dermatan sulfate is lacking due to the cell line being engineered not to have a functional proteoglycan synthesis pathway.

5. The use according to claim 4, wherein the cell line is engineered to lack an active beta-1,3-glucuronyltransferase 3 (B3GAT3) protein.

6. The use according to claim 5, wherein the active B3GAT3 protein is lacking due to the cell line being genetically engineered to lack expression of the B3GAT3 protein.

7. The use according to any one of claims 1 to 6, wherein the cell line is derived from a cell line selected from the group consisting of human embryonic kidney 293T (HEK293T) cell line, human embryonic kidney 293 (HEK293) cell line, HeLa cell line, D-17 cell line, HT1080 cell line, TE671 cell line, MV-1-Lu cell line and HOS cell line.

8. The use according to claim 7, wherein the cell line is derived from a HEK293T cell line.

9. The use according to claim 8, wherein the cell line is a HEK293T cell line in which the *B3GAT3* gene encoding the B3GAT3 protein is inactivated.

10. The use according to claim 9, wherein the cell line is deposited under number DSM ACC3355 or DSM ACC3356.

11. The use according to any one of claims 1 to 10, wherein the virus is selected from the group consisting of a retrovirus, a spumavirus, a lentivirus, an adenovirus, an adeno-associated virus, a herpesvirus, a human papillomavirus (HPV), a hepatitis virus, a Dengue virus (DENV), a Japanese encephalitis virus (JEV), a yellow fever virus (YFV), a togavirus, a filovirus, an orthobunyavirus and a rabies virus (RABV).

12. A method for producing recombinant virus particles, the method comprising the steps of:
(a) transfecting a plurality of cells of a mammalian packaging cell line engineered to lack cell surface expression of heparan sulfate with
(i) a transfer vector, and
(ii) at least one viral packaging plasmid;
thereby obtaining a plurality of producer cells;
(b) culturing the producer cells in a medium, thereby obtaining the virus particles in the medium; and
(c) separating the medium from the producer cells.

13. The method according to claim 12, wherein step (a) is performed by using a non-cationic transfection reagent, preferably calcium phosphate.

14. A recombinant virus particle obtainable by the method of claim 12 or 13.

15. A mammalian packaging cell line deposited under number DSM ACC3355 or DSM ACC3356.
